Europäisches Patentamt

European Patent Office

Office européen des brevets

(19)

(11) Publication number: 0 396 505
A2

## EUROPEAN PATENT APPLICATION

(21) Application number: 90810326.0

(22) Date of filing: 26.04.90

(51) Int. Cl.⁵: **C12P 21/08, C12N 5/12,**
**G01N 33/577, A61K 39/395**

(30) Priority: **04.05.89 GB 8910263**

(43) Date of publication of application:
**07.11.90 Bulletin 90/45**

(84) Designated Contracting States:
**AT BE CH DE DK ES FR GB GR IT LI LU NL SE**

(71) Applicant: **CIBA-GEIGY AG**
**Klybeckstrasse 141**
**CH-4002 Basel(CH)**

(72) Inventor: **Heusser, Christoph, Dr.**
**Im Bertschenacker 21**
**CH-4103 Bottmingen(CH)**
Inventor: **Wagner, Kathrin**
**Sundgauerstrasse 12**
**CH-4055 Basle(CH)**
Inventor: **Bews, John Patrick Anthony**
**Hinter Gärten 11**
**CH-4125 Riehen(CH)**

(54) **Monoclonal antibodies specific for an immunoglobulin isotype.**

(57) The invention concerns monoclonal antibodies directed against isotypic determinants of immunoglobulin E (IgE), processes for the preparation of said antibodies, continuous cell lines secreting said antibodies and processes for the preparation of said cell lines. The monoclonal antibodies are useful for the determination of IgE and IgE-producing cells and in the treatment and prophylaxis of allergy.

EP 0 396 505 A2

## Monoclonal antibodies specific for an immunoglobulin isotype

The invention concerns monoclonal antibodies directed against isotypic determinants of immunoglobulin E (IgE), especially of murine or human IgE, and derivatives thereof. The monoclonal antibodies may be chimeric or bispecific. They recognize free IgE and IgE expressed on the surface of IgE positive B cells, but do not recognize IgE bound to cells bearing $Fc_\epsilon$ receptors I or II and do not trigger mediator release. Processes for the preparation of these antibodies and their derivatives, continuous cell lines secreting the antibodies and processes for the preparation of the cell lines are also subjects of the invention. The monoclonal antibodies and/or their derivatives are useful for the determination of IgE and IgE-producing cells and in the treatment and prophylaxis of allergy. The invention also concerns test kits and pharmaceutical compositions comprising the antibodies and/or their derivatives.

Background of the invention

Allergy is a hypersensitive state induced by an exaggerated immune response to a foreign agent (the allergen). Immediate (type I) hypersensitivity, characterized by allergic reactions immediately following contact with the allergen, is mediated via B cells and is based on antigen-antibody reactions, whereas delayed hypersensitivity is mediated via T cells and based on mechanisms of cellular immunity. In recent years, the term "allergy" has become more and more synonymous with type I hypersensitivity.

Immediate hypersensitivity is based on the production of antibodies of the immunoglobulin class E (IgE antibodies) by B cells which upon confrontation with the allergen differentiate into antibody secreting plasma cells. The IgE induced reaction is a local event occurring at the site of the allergen's entry into the body, i.e. at mucosal surfaces and/or at local lymph nodes. Locally produced IgE will first sensitize local mast cells, i.e. IgE antibodies bind with their constant regions to $Fc_\epsilon$ receptors on the surface of the mast cells, and then "spill-over" IgE enters the circulation and binds to receptors on both circulating basophils and tissue-fixed mast cells throughout the body. When the bound IgE is subsequently contacted with the allergen, the $Fc_\epsilon$ receptors are crosslinked by binding of the allergen and the cells are caused to degranulate and release a number of anaphylactic mediators such as histamine, prostaglandins, leukotrienes etc.. It is the release of these substances which is responsible for the clinical symptoms typical of immediate hypersensitivity, namely contraction of smooth muscle in the respiratory tract or the intestine, the dilation of small blood vessels and the increase in their permeability to water and plasma proteins, the secretion of mucus resulting e.g. in rhinitis, atopic excema and asthma, and the stimulation of nerve endings in the skin resulting in itching and pain.

In addition, the reaction upon second contact with the allergen is intensified because some B cells form a "memory pool" of surface IgE positive B cells ($sIgE^+$ B cells) after the first contact with the allergen by expressing IgE on the cell surface.

Current treatment of allergy focuses on two approaches. One treats only the symptoms of allergy, for example by utilizing drugs which inhibit the mediators such as antihistamines or which are immunosuppressive such as cytostatics or corticosteroids. This approach entails repeated doses and can involve undesirable side effects. Another therapeutic approach named desensitization is intended to treat the underlying conditions of allergy. The goal of desensitization is to achieve improved tolerance of the patient to a specific allergen by administering initially small, subsequently increasing doses of the offending allergen over a period of time. This approach can involve considerable discomfort, presupposes the identification and availability of the allergen(s) and requires a large number of visits to a doctor. Moreover, the allergen to which the patient is hypersensitive cannot always be identified, making desensitization treatment impossible. Furthermore, the treatment is not without risk. Neither of the above approaches can protect against the development of a hypersensitive state.

A promising alternative would be a therapeutical treatment which inhibits allergic reactions by downregulating the IgE immune response, which is the earliest event in the induction of allergy and provides for the maintenance of the allergic state, without effecting the response of other antibody classes, thus leading to both an immediate and a long lasting effect on allergic symptoms. Such a treatment might be based on the application of antibodies, e.g. monoclonal antibodies, which are IgE isotype-specific and are thus capable of binding IgE. In addition, such antibodies should react with surface IgE positive B cells which lead to IgE producing plasma cells, so that they could be used to functionally eliminate those B cells. However, antibodies to IgE in principle may also induce mediator release from IgE sensitized mast cells by crosslinking the $Fc_\epsilon$ receptors, thus antagonizing the beneficial effect exerted on the serum IgE and $sIgE^+$ B

2

cell level. In consequence, antibodies applicable in therapy of allergy must not be capable of reacting with IgE bound on sensitized mast cells and basophils, but should retain the capability to recognize sIgE[+] B cells.

IgE isotype-specific antibodies have been described by several research groups. Conrad et al. (Int. Archs. Allergy Appl. Immun. 70, 352, 1983) for example describe murine anti-rat IgE monoclonal antibodies which, however, do not interfere with binding of IgE to must cells. Chrétien et al. (J. Immunol. 141, 3128, 1988) disclose murine anti-human IgE monoclonal antibodies, two of which are capable of inhibiting the binding of IgE to $Fc_\epsilon$ receptor bearing cells. However, all of the described antibodies induce mediator release.

## Object of the invention

It is an object of this invention to provide monoclonal antibodies directed against isotopic determinants of IgE which
• inhibit the binding of IgE to cells bearing Fcf receptors I or II,
• recognize and bind IgE expressed on the surface of surface IgE positive B cells (sIgE[+] B cells),
• do not recognize and bind IgE bound on the surface of cells bearing $Fc_\epsilon$ receptors I or II, for example sensitized mast cells and basophils, and thus
• do not trigger mediator release from sensitized cells.

The monoclonal antibodies of the invention have these characteristics and thus overcome the drawbacks of the IgE isotype-specific antibodies known in the art. They are capable of neutralizing formed IgE antibodies and depleting the population of sIgE[+] B cells without triggering mediator release from already sensitized cells. They are therefore extremely useful for the treatment as well as prophylaxis of allergy.

Surprisingly, it was found that the monoclonal antibodies of the invention have an inhibitory effect on the formation of IgE in the immune response while not affecting the immune response of other antibody classes. In consequence, they are especially good therapeutic and prophylactic agents since they suppress the IgE response directly from the beginning by inhibiting the formation of IgE.

Thus, it is an object of the invention to provide a method of treating and preventing allergy that
• treats the underlying causes of allergy instead of the symptoms,
• does not require identification of specific allergen(s),
• is not limited to treating the hypersensitivity response to particular allergen(s),
• does not require a large number of repeated doses, and
• induces a long-lasting effect.

It is also desirable to produce monoclonal antibodies with the desired specificity which are bispecific, thus being able to intensify the therapeutic effect by choosing an appropriate second antibody specificity, or which are chimeric, i.e. possess human constant regions, and are therefore less immunogenic and do not induce anaphylactic reactions upon application. The constant regions can be chosen to optionally mediate endogenous effector functions.

## Description of the invention

The invention concerns monoclonal antibodies directed against isotypic determinants of immunoglobulin E (IgE) which inhibit the binding of free IgE to cells bearing $Fc_\epsilon$ receptors I or II and recognize IgE expressed on the surface of surface IgE positive B cells, but do not recognize IgE bound to the surface of cells bearing $Fc_\epsilon$ receptors I or II and thus do not trigger mediator release from such cells, and derivatives thereof retaining the specificity of the antibody from which they are derived. Preferred are monoclonal antibodies of the invention and derivatives thereof which do not recognize IgE bound to the surface of $Fc_\epsilon$ receptor I bearing mast cells and basophils and do not trigger anaphylactic mediator release from such cells. Also preferred are monoclonal antibodies and derivatives thereof with the desired specificity which in addition specifically inhibit IgE formation in the immune response.

The monoclonal antibodies and their derivatives of the invention recognize antigenic determinants on the $\epsilon$ heavy chain common to immunoglobulins of class IgE, i.e. they react with IgE molecules of different specificities but do not react with immunoglobulins of other isotypes or with light chains. The monoclonal antibodies and derivatives thereof according to the invention neutralize free IgE antibodies, i.e. they bind the free IgE antibodies in such a manner that binding of IgE to cells bearing $Fc_\epsilon$ receptors I or II is inhibited.

$Fc_\epsilon$ receptors I are high affinity $Fc_\epsilon$ receptors (dissociation constant $K_D$ approximately $10^{10}$) and are found on mast cells and basophils. $Fc_\epsilon$ receptors II are low affinity $Fc_\epsilon$ receptors ($K_D$ approximately $10^6$) and are found on monocytes, alveolar macrophages, eosinophils, and certain T and B cells (surface $\mu\delta^+$ activated B cells). In addition, the monoclonal antibodies and their derivatives of the invention are capable of functionally eliminating surface IgE positive ($sIgE^+$) B cells since they recognize IgE expressed on the surface of such B cells, thus affecting the "allergic memory". However, they do not recognize IgE bound to the surface of cells bearing $Fc_\epsilon$ receptors I or II, in particular sensitized mast cells and basophils, i.e they react with epitopes of IgE which are hidden when IgE is bound on the surface of such cells. As a result of this specificity, the monoclonal antibodies and derivatives thereof of the invention do not effect crosslinking of $Fc_\epsilon$ receptors and thus do not induce mediator release from cells bearing $Fc_\epsilon$ receptors, e.g. do not cause degranulation of mast cells and basophils and anaphylactic mediator release. Preferred are monoclonal antibodies and derivatives thereof with the abovedescribed specificity which specifically inhibit IgE formation in the immune response, i.e. which prevent the differentiation of B cells to IgE producing cells.

The ability of the monoclonal antibodies of the invention to inhibit the binding of IgE to $Fc_\epsilon$ receptor bearing cells can for example be determined in a competitive radioimmunoassay wherein e.g. must cells, for instance in suspension or bound to a suitable carrier, are incubated with radioactively labelled IgE, e.g. $^{125}I$-IgE, and the monoclonal antibody of the invention to be tested, and the amount of IgE bound to the cells is determined by measuring the radioactivity. The ability of the monoclonal antibodies of the invention to inhibit the binding of IgE to $Fc_\epsilon$ receptor II bearing cells can for example be detected by determining the inhibition of the agglutination of IgE-coated latex beads with e.g. $Fc_\epsilon$ receptor II bearing RPMI 8866 cells.

Staining experiments demonstrate that the monoclonal antibodies of the invention recognize $sIgE^+$ B cells but do not recognize IgE bound to the surface of e.g. mast cells and basophils.

Mediator release induced by the monoclonal antibodies of the invention can for example be determined by incubating e.g. mast cells with IgE, adding the monoclonal antibody of the invention and measuring the amount of histamine released by the cells, for example in a radioimmunoassay.

The inhibitory effect of the monoclonal antibodies of the invention on the IgE formation in the immune response can for example be determined in an in vitro or in vivo immune response assay wherein either B cells in culture or entire animals are challenged with an allergen and IgE antibodies formed are measured, e.g. by an enzyme immunoassay or a radioimmunoassay, or IgE-producing cells are determined, for example by the cell plaque forming assay described hereinbelow.

The monoclonal antibodies of the invention may be of immunoglobulin class IgG, IgM, IgA or IgD, preferentially of class IgG, most preferably of class IgG1. The different classes and subclasses of immunoglobulins, which are primarily determined by the carboxy-terminal constant region segments of the H-chains, are involved in different effector functions (complement binding, stimulation of phagocytosis, neutrophil activation etc.). In consequence, by using a monoclonal antibody of the invention of a specific Ig class, the role of the antibody in the immune response can be chosen depending on the desired effect. For example, IgG1 antibodies play a major role in antibody-dependent cell-mediated cytotoxicity (ADCC), a reaction in which special effector cells with cytotoxic potential bind to the antibody $Fc_\gamma$ receptors and lyse the target cells to which the antibodies are directed. Thus, monoclonal antibodies of the invention which are of Ig subclass IgG1 might be used to specifically deplete the $sIgE^+$ B cell population by binding to $sIgE^+$ B cells and attracting cytotoxic cells which kill the $sIgE^+$ B cells.

Preferred are monoclonal antibodies, preferentially rat monoclonal antibodies, and derivatives thereof of the invention directed against isotypic determinants of murine IgE. Most preferred is the rat monoclonal antibody with the designation MAb 1-5 which is specific for murine IgE, and derivatives thereof.

Further preferred are monoclonal antibodies, preferentially mouse or rat monoclonal antibodies, and derivatives thereof of the invention directed against isotypic determinants of human IgE. Most preferred is the mouse monoclonal antibody with the designation MAb F1-43-17 which is specific for human IgE, and derivatives thereof.

Also preferred are chimeric animal/human monoclonal antibodies and derivatives thereof according to the invention directed against isotypic determinants of human IgE. Chimeric monoclonal antibodies of this invention consist of chimeric heavy and light immunoglobulin chains. Each chimeric chain is a contiguous polypeptide that has a nonhuman variable region with the desired specificity and a human constant region. The chimeric heavy and light chains are associated to form a molecule with a functional antigen binding region. Most preferred are chimeric monoclonal antibodies according to the invention directed against isotypic determinants of human IgE which consist of murine variable regions and human constant regions, and derivatives thereof.

The heavy chain constant region for the chimeric immunoglobulins can be selected from any of the isotypes alpha, delta, gamma or mu. Heavy chains of various subclasses such as the IgG subclasses 1-4 can be used. The different classes and subclasses of heavy chains are involved in different effector functions and thus, by choosing the type of heavy chain constant region, chimeric antibodies with the desired effector function can be produced. The light chains can have either a kappa or lambda constant chain.

Also preferred are bispecific monoclonal antibodies comprising two different antigen binding portions, one derived from a monoclonal antibody according to the invention directed against isotypic determinants of human IgE and the second derived from an antibody with a different specificity. For example, the second specificity can be for an agent such as a cytostatic or cytotoxic drug to achieve drug delivery to B cells expressing surface IgE (sIgE$^+$ B cells). Suitable cytostatic or cytotoxic substances are those described for monoclonal antibody derivatives hereinbelow. The second specificity can also be derived from another monoclonal antibody of the invention which recognizes a different epitope of human IgE than the first antibody, thus facilitating the formation of immune complexes and complement fixation.

Derivatives of a monoclonal antibody of the invention retain the specificity of the antibody from which they are derived, i.e. they retain the characteristic binding pattern of the parent antibody to IgE. Examples of such derivatives are monoclonal antibody fragments, conjugates of the monoclonal antibodies with an enzyme, a fluorescent marker, a chemiluminescent marker, a metal chelate, a cytostatic or cytotoxic substance, avidin, biotin or the like, or radioactively labelled monoclonal antibodies.

Antibody fragments of the invention are for example the univalent fragments Fab or Fab′ (Fab: fragment antigen binding) or the divalent fragment F(ab′)$_2$.

Enzymes used for antibody conjugates of the invention are, for example horseradish peroxidase, alkaline phosphatase, $\beta$-D-galactosidase, glucose oxidase, glucoamylase, carbonic anhydrase, acetylcholinesterase, lysozyme, malate dehydrogenase or glucose-6-phosphate dehydrogenase.

Fluorescent markers conjugated with monoclonal antibodies of the invention can be fluorescein, fluorochrome, rhodamine, and the like.

Chemiluminescent markers are e.g. acridinium esters of luminol.

In such conjugates the antibody is bound to the conjugation partner directly or by way of a spacer or linker group.

Examples for metal chelators are ethylenediaminetetraacetic acid (EDTA), diethylenetriaminepentaacetic acid (DPTA), 1,4,8,11-tetraazatetradecane, 1,4,8,11-tetraazatetradecane-1,4,8,11-tetraacetic acid, 1-oxa-4,7,12,15-tetraazaheptadecane-4,7,12,15-tetraacetic acid, or the like.

Cytostatics, applicable in connection with the monoclonal antibodies of the invention, are, inter alia, alkylating substances, such as mechlorethamine, triethylenephosphoramide, cyclophosphamide, ifosfamide, chlorambucil, busulfan, melphalan or triaziquone, also nitrosourea compounds, such as carmustine, lomustine, or semustine. Also used are antimetabolites, such as methotrexate, mercaptopurine, cytarabine, fluorouracil, floxuridine, or ftorafur. A further group of cytostatics includes vinblastine and vincristine, as well as certain antibiotics, such as actinomycin-D, daunorubicin (daunomycin), doxorubicin, mithramycin, streptonigrin, mitomycin and bleomycin. Further suitable cytostatics are, inter alia, procarbacine, hydroxyurea, L-asparaginase, dacarbazine, mitotane, estramustine, or podophyllotoxin. Further cytostatic agents are hormones and hormone antagonists, such as corticosteroids, e.g. prednisone, progestins, e.g. hydroxyprogesterone or medroprogesterone, estrogens, e.g. diethylstilbestrol, antiestrogens, e.g. tamoxifen, androgens, e.g. testosterone, and aromatase inhibitors, e.g. aminogluthetimide.

Conjugates of monoclonal antibodies of the invention with cytotoxic substances can contain either the intact toxin or the A-chain derived from it. Toxins suitable for antibody-coupling are, among others, several lectins, such as ricin or abrin, or diphtheria toxin A, and the like.

Radioactively labelled monoclonal antibodies of the invention contain e.g. radioactive iodine ($^{123}$I, $^{125}$I, $^{131}$I), yttrium ($^{90}$Y), technetium ($^{90m}$Tc), or the like.

The monoclonal antibodies and derivatives thereof according to the invention are prepared by processes known per se wherein cells of a continous cell line as defined further below secreting the desired monoclonal antibodies are multiplied according to known methods in vitro or in vivo and, when required, the obtained monoclonal antibodies are isolated and/or converted into derivatives thereof.

Multiplication in vitro is carried out in suitable culture media, which are the customary standard culture media, for example Dulbecco's Modified Eagle Medium (DMEM) or RPMI 1640 medium, optionally replenished by a mammalian serum, e.g. fetal calf serum, or trace elements and growthsustaining supplements, e.g. feeder cells such as normal mouse peritoneal exudate cells, spleen cells, bone marrow macrophages, 2-aminoethanol, insulin, transferrin, low density lipoprotein, oleic acid, or the like.

In vitro production provides relatively pure antibody preparations and allows scale-up to give large amounts of the desired antibodies. Techniques for mammalian cell cultivation under tissue culture conditions are known in the art and include homogeneous suspension culture, e.g. in an airlift reactor or in a continuous stirrer reactor, or immobilized or entrapped cell culture, e.g. in hollow fibres, microcapsules, on agarose microbeads or ceramic cartridges.

For isolation of the monoclonal antibodies, the immunoglobulins in the culture supernatants are first concentrated, e.g. by precipitation with ammonium sulphate, dialysis against hygroscopic material such as polyethylene glycol (PEG), filtration through selective membranes, or the like. If necessary and/or desired, the concentrated antibodies are purified by the customary chromatography methods, for example gel filtration, ion-exchange chromatography, chromatography over DEAE-cellulose or protein A, or immunoaffinity chromatography.

Large quantities of the desired monoclonal antibodies can also be obtained by multiplying the cells in vivo. For this purpose, cell clones from a histocompatible and/or tolerated Ig-producing cell line are injected into syngeneic mammals to cause growth of antibody-producing tumors. Optionally, the animals are primed with a hydrocarbon, especially mineral oils such as pristane (tetramethylpentadecane), prior to the injection. After 1-3 weeks, the antibodies are isolated from the body fluids of those mammals. For example, hybridoma cells derived from Balb/c mice that produce the desired monoclonal antibodies are injected intraperitoneally into Balb/c mice optionally pre-treated with pristane, and, after one to two weeks, ascitic fluid is taken from these animals. The desired monoclonal antibodies are isolated therefrom by conventional methods as given above.

Fragments of monoclonal antibodies, for example Fab, Fab′ or F(ab′)$_2$ fragments, can be obtained from the antibodies prepared as described above by methods known per se, e.g. by digestion with enzymes such as papain or pepsin and/or cleavage of disulfide bonds by chemical reduction.

Conjugates of monoclonal antibodies of the invention are prepared by methods known in the art, e.g. by reacting a monoclonal antibody prepared as described hereinbefore with an enzyme in the presence of a coupling agent, e.g. glutaraldehyde, periodate, N,N′-o-phenylenedimaleimide, N-(m-maleimidobenzoyloxy)-succinimide, N-(3-[2′-pyridyldithio]-propionoxy)-succinimide, N-ethyl-N′-(3-dimethylaminopropyl)-carbodiimide or the like. Conjugates with avidin are prepared likewise. Conjugates with biotin are prepared e.g. by reacting monoclonal antibodies with an activated ester of biotin such as the biotin N-hydroxysuccinimide ester. Conjugates with fluorescent or chemiluminescent markers are prepared in the presence of a coupling agent, e.g. those listed above, or by reaction with an isothiocyanate, preferably fluorescein-isothiocyanate. Conjugates of the monoclonal antibodies of the invention with cytostatic/cytotoxic substances and metal chelates are prepared in an analogous manner.

Monoclonal antibodies radioactively labelled with iodine ($^{123}$I, $^{125}$I, $^{131}$I) are obtained from the monoclonal antibodies according to the Invention by iodination known per se, for example with radioactive sodium or potassium iodide and a chemical oxidizing agent, such as sodium hypochlorite, chloramine T or the like, or an enzymatic oxidizing agent, such as lactoperoxidase, glucose oxidase and glucose. Chimeric monoclonal antibodies according to the invention are coupled to yttrium ($^{90}$Y) for example by diethylenetriaminepentaacetic acid (DTPA) chelation. Technetium-99m labelled chimeric antibodies are prepared by ligand exchange processes, for example by reducing pertechnate (TcO$_4$$^-$) with stannous ion solution, chelating the reduced technetium onto a Sephadex column and applying the antibodies to this column, or by direct labelling techniques, e.g. by incubating pertechnate, a reducing agent such as SnCl$_2$, a buffer solution such as a sodium potassium phthalate-solution, and the antibodies.

The invention further concerns continuous cell lines. In one embodiment of the invention, such cell lines are hybridoma cell lines secreting anti-IgE monoclonal antibodies of the invention with the desired specificity.

In particular, the invention concerns hybridoma cell lines which are hybrids of myeloma cells and B lymphocytes of a mammal immunized with IgE. Preferentially these cell lines are hybrids of mouse myeloma cells and B lymphocytes of rats immunized with murine IgE. Especially preferred is the hybridoma cell line with the designation 654-1-5, which was deposited at the European Collection of Animal Cell Cultures (ECACC), PHLS Centre for Applied Microbiology & Research, Porton Down, Salisbury, Wilts. SP4 OJG, U.K., under the number ECACC 89041902 on April 19, 1989. Also preferred are hybridoma cell lines which are hybrids of mouse myeloma cells and B lymphocytes of a syngeneic mouse immunized with human IgE. Most preferred is the hybridoma cell line with the designation F1-43-17 which was deposited at the European Collection of Animal Cell Culture under the number ECACC 90032210 on March 22, 1990.

The hybridoma cell lines of the invention are genetically stable, secrete the monoclonal antibodies of the invention with constant specificity and may be kept in deep-frozen cultures and reactivated by thawing and optional re-cloning.

The invention also concerns a process for the preparation of hybridoma cell lines secreting the monoclonal antibodies of the invention wherein a suitable mammal is immunized with IgE, antibody-producing cells of this mammal are fused with cells of a continuous cell line, the hybrid cells obtained in the fusion are cloned, and cell clones secreting the desired antibodies are selected.

The immunoglobulin E used as antigen for the immunization may be murine or human IgE, respectively. It is preferable to use a mixture of diverse antibodies, especially monoclonal antibodies, of class IgE with different specificities to ensure that IgE isotype-specific antibodies are elicited which are directed against the $\epsilon$ heavy chain. Such IgE antibodies are for example commercially available or may be obtained from cell culture collections or depository institutions. Human IgE may also be obtained from patients with IgE myeloma.

The antigen, i.e. marine or human IgE, may be mixed with adjuvants, i.e. agents that will further increase the immune response, for the immunization procedure. Possible adjuvants are Freund's complete adjuvant (emulsion of mineral oil, water, and mycobacterial extracts), Freund's incomplete adjuvant (emulsion of water and oil only), aluminium hydroxide gels, or the like.

The antigen is used to immunize suitable mammals which recognize it as a foreign molecule, especially rats when the antigen is marine IgE, or mice or rats, preferentially mice, when the antigen is human IgE.

The routes of immunization include, among others, intradermal, subcutaneous, intramuscular, intraperitoneal, intravascular and intracranial injections. Since high antibody titers are desired, a series of injections is commonly given. The immunization is for example performed by injecting the antigen, optionally mixed with incomplete or complete Freund's adjuvant, three to eight times parenterally, e.g. intraperitoneally and/or subcutaneously, in amounts of around 10-150 $\mu$g into RA 25 rats, Balb/c mice or Biozzi mice at intervals of 1-3 weeks, followed by a booster injection of about 5-50 $\mu$g 1-5 days prior to sacrificing the animals.

Antibody-producing cells of the immunized mammals, preferably lymphoid cells such as spleen lymphocytes, taken for example 1-5 days after the final booster injection, are fused with the cells of a continuous cell line, i.e. a continuously replicating cell clone which confers this replication ability to the hybrid cells resulting from the fusion. An example for such a cell line is a tumor cell line (myeloma) which does not itself produce immunoglobulins or fragments thereof but has the potential to produce and secrete large amounts of antibody, and which carries a genetic marker so that the hybrid cells can be selected against non-fused parent cells. Several suitable myeloma cell lines are known in the art. Preferred are myeloma cell lines lacking the enzyme hypoxanthine guanine phosphoribosyl transferase (HGPRT) or the enzyme thymidine kinase (TK),which therefore do not survive in a selective culture medium containing hypoxanthine, aminopterin and thymidine (HAT medium). Particularly preferred are myeloma cells and derived cell lines that do not survive in HAT medium and do not secrete immunoglobulins or fragments thereof, such as the cell lines PAI, X63-Ag8.653 or Sp2/O-Ag14.

The fusion is performed in the presence of a fusion promoter, for example Sendai virus or other paramyxo viruses, optionally in UV-inactivated form, or chemical fusogens such as calcium ions, surface-active lipids, e.g. lysolecithin, or polyethylene glycol (PEG). Preferentially, the myeloma cells are fused with a three- to twentyfold excess of spleen cells from immunized mammals in a solution containing about 30 % to about 60 % polyethylene glycol of a molecular weight between 1000 and 4000.

After the fusion, the cells are resuspended and cultivated in a selective medium, for example HAT medium. In this medium, only hybridoma cells will survive, because they combine the ability to grow and replicate in vitro like the parent myeloma cells and the missing HGPRT or TK genes essential for the survival in the HAT medium inherited from the antibody-producing spleen cells of the immunized mammals.

Suitable culture media for the expansion of hybridoma cells are the standard culture media, such as Dulbecco's modified Eagle medium (DMEM), minimum essential medium, RPMI 1640 medium and the like, optionally replenished by a mammalian serum, e.g. 10 to 15 % fetal calf serum. Preferentially, feeder cells, e.g. normal mouse peritoneal exudate cells, spleen cells, bone marrow macrophages, or the like, are added at the beginning of the cell growth immediately after the fusion step to nourish the hybridoma cells and support their growth, especially where cell densities are low, by providing growth factors and the like. If phagocytic cells such as macrophages or monocytes are used, they can perform a helpful service in cleaning up the debris of dead myeloma cells always found after aminopterin treatment. The culture media are supplemented with selective medium in order to prevent myeloma cells from overgrowing the hybridoma cells.

The hybridoma cell culture supematants are screened for the desired monoclonal antibodies, preferentially with an enzyme immunoassay or a radioimmunoassay. Such an immunoassay is for example an enzyme-linked immunosorbent assay (ELISA) wherein a suitable carrier, e.g. plastic microtitre plates, is coated with immunoglobulins and/or myeloma proteins of all immunoglobulin classes and subclasses and incubated with the hybridoma cell culture supernatants to be tested. Bound monoclonal antibodies are detected by incubation with an enzyme-labelled antibody recognizing the anti-IgE antibodies in the cell supernatant, e.g. an alkaline phosphatase-labelled antibody against immunoglobulins of the animal species used for immunization, and by addition of an appropriate enzyme substrate solution, e.g. p-nitrophenylphosphate. The enzyme substrate reaction results, for example, in a color change which can be observed by eye or with optical measuring devices. The hybridomas producing high titres of antibodies which react with IgE but not with immunoglobulins of other classes are selected for further studies.

Positive hybridoma cells are cloned, e.g. by limiting dilution or in soft agar, preferentially twice or more. Optionally, hybridoma cells are passaged through animals, e.g. mice, by intraperitoneal injection and harvesting of ascites, which stabilizes hybridomas and improves growth characteristics. The cloned cell lines may be frozen in a conventional manner.

The invention also concerns continuous cell lines which are transfectoma cell lines secreting chimeric animal/human monoclonal antibodies, preferentially chimeric monoclonal antibodies consisting of murine vale regions and human constant regions, according to the invention. By definition, transfectoma cell lines are continuous cell lines such as immortalized mammalian cell lines, e.g. lymphoma, myeloma, hybridoma, trioma or quadroma cell lines, transfected with nucleic acid constructs encoding the chimeric light and/or heavy chains of the chimeric animal/human monoclonal antibodies of the invention.

Furthermore, the invention concerns a process for the preparation of transfectoma cell lines secreting chimeric animal/human monoclonal antibodies of the invention wherein cells of a continuous cell line are transfected with nucleic acid, preferably DNA, constructs encoding the chimeric animal/human light and/or heavy chains of the desired chimeric monoclonal antibodies, the transfected cells obtained are cloned, and cell clones secreting the desired chimeric monoclonal antibodies are selected.

A chimeric DNA construct for each the light and the heavy chains of the chimeric monoclonal antibodies of the invention comprise a first DNA segment, e.g. a functionally rearranged gene, encoding at least the functional portion of the variable regions of a nonhuman, preferably murine, antibody with the desired specificity linked to a second DNA segment encoding at least a portion of the constant regions of a human antibody.

The DNA segment encoding the nonhuman variable regions can for example be obtained from a hybridoma cell line producing nonhuman antibodies with the desired specificity, either by isolation of genomic DNA or by preparation of cDNA from isolated mRNA. Screening of the genomic DNA or cDNA for the functionally rearranged variable region can be accomplished with the use of appropriate DNA probes of known nucleotide sequence, for example synthetic DNAs,cDNAS derived from mRNA coding for the desired immunoglobulin or genomic DNA fragments comprising e.g. adjacent conserved variable or constant regions. Identification and confirmation of the correct clones can then be achieved by DNA sequencing of the cloned genes and comparison of the sequence to the corresponding sequence of the full length, properly spliced mRNA.

The DNA segment encoding the human constant regions can be obtained from a genomic library derived from suitable human tissue. The genomic DNA library is screened with DNA probes and the desired clones are identified as described above.

The joining of the DNA segments in order to produce chimeric gene constructs is performed in accordance with conventional techniques, for example by blunt- or staggered-end ligation, restriction enzyme digestion to provide for appropriate cohesive termini, filling in cohesive ends as appropriate, alkaline phosphatase treatment to avoid undesirable joining, and ligation with appropriate ligases.

The chimeric DNA constructs are assembled in or inserted into replicable hybrid vectors. Vectors typically perform two functions in collaboration with compatible host cells. One function is to facilitate the cloning of the nucleic acid that encodes the chimeric immunoglobulin chains, i.e. to produce usable quantities of the nucleic acid (cloning vectors). The other function is to provide for replication and expression of the chimeric gene constructs in a suitable host, either by maintenance as an extrachromosomal element or by integration into the host chromosome (expression vectors). A cloning vector comprises the chimeric gene constructs as described above, an origin of replication or an autonomously replicating sequence, dominant marker sequences and, optionally, signal sequences and additional restriction sites. An expression vector additionally comprises expression control sequences essential for the transcription and translation of the chimeric genes.

An origin of replication or an autonomously replication sequence is provided either by construction of the vector to include an exogeneous origin such as derived from Simian virus 40 (SV 40) or another viral source, or by the host cell chromosomal mechanisms.

The markers allow for selection of host cells which contain the vector. Selection markers include genes which confer resistance to heavy metals such as copper or to antibiotics such as geneticin (G-418) or hygromycin, or genes which complement a genetic lesion of the host cell such as the absence of thymidin kinase, hypoxanthine phosphoryl transferase, dihydrofolate reductase or the like.

Signal sequences may be for example a presequence or secretory leader directing the secretion of the antibody, splice signals, or the like.

As expression control sequences, the vector DNA comprises a promoter, sequences necessary for the initiation and termination of transcription and for stabilizing the mRNA and, optionally, enhancers and further regulatory sequences. A wide variety of promoting sequences may be employed, depending on the nature of the host cell. Promoters suitable for mammalian host cells are obtained from viruses such as Simian virus 40 (SV 40), Rous sarcoma virus (RSV), adenovirus 2, bovine papilloma virus (BPV), papovavirus BK mutant (BKV), or mouse and human cytomegalovirus (CMV). Alternatively, the vectors may comprise promoters from mammalian expression products, such as actin, collagen, myosin etc., or the native promoter and control sequences which are normally associated with the immunoglobulin gene sequences. Sequences necessary for the initiation and termination of transcription and for stabilizing the mRNA are commonly available from the noncoding 5' regions and 3' regions, respectively, of viral and eukaryotic cDNAS, e.g. from the expression host. Enhancers are transcription-stimulating DNA sequences of viral origin, e.g. derived from Simian virus, polyoma virus, bovine papilloma virus or Moloney sarcoma virus, or of genomic, especially murine origin.

The various DNA segments of the vector DNA are operationally linked, i.e. they are contiguous and placed into a functional relationship with each other.

Separate vectors may be constructed comprising either the chimeric L-chain gene construct or the chimeric H-chain gene construct, or, alternatively, both L- and H-chain gene constructs are cloned into the same vector.

Examples of preferred vectors are those derived from the plasmid pSV in which transcription is controlled by the SV 40 early promoter, especially pSV2gpt carrying the xanthine-guanine phosphoribosyl transferase gene, or pSV2neo carrying the phosphotransferase gene.

Suitable host cells for expressing the vectors have to be capable of culture in vitro and have to be of higher eukaryotic origin to provide a suitable environment for the production of active antibodies, since the biosynthesis of functional tetrameric antibody molecules requires correct nascent polypeptide chain folding, glycosylation, and assembly. Examples of suitable host cells are immortalized mammalian cell lines, such as lymphoma, myeloma, hybridoma, trioma or quadroma cell lines, e.g. the myeloma cell lines PAI, Sp2/O-Ag14 or X63-Ag8.653.

These host cells are transfected with the chimeric L-chain gene construct alone, with the chimeric H-chain gene construct alone, or with both, either sequentially or simultaneously transferred with the help of two separate vectors or in a one-step procedure by using a double-construct (L-chain/ H-chain) vector as indicated hereinbefore.

Transfection is carried out by conventional techniques, such as calcium phosphate precipitation, microinjection, protoplast fusion, electroporation, i.e. introduction of DNA by a short electrical pulse which transiently increases the permeability of the cell membrane, or the like.

After the transfection procedure, transfected cells are identified and selected by cultivation in a selective medium chosen depending on the nature of the selective marker, for example the media described hereinbefore for selection of hybridoma cells, in which only transfected cells survive.

The invention also concerns continuous cell lines, e.g. immortalized mammalian cell lines such as hybridoma, trioma or quadroma cell lines, secreting bispecific monoclonal antibodies of the invention.

Furthermore, the invention concerns a process for the preparation of continuous cell lines secreting bispecific monoclonal antibodies of the invention wherein cells producing heavy and/or light chains of an anti-IgE monoclonal antibody of the invention with the desired sepcificity and cells producing heavy and/or light chains of a monoclonal antibody with a different specificity are fused, the hybrid cells obtained in the fusion are cloned, and cell clones secreting the desired bispecific monoclonal antibodies are selected.

Fusion can for example be performed by fusing a hybridoma producing monoclonal antibodies of the invention with lymphoid cells of a mammal immunized with an antigen determining the second specificity of the bispecific monoclonal antibody, with a myeloma secreting myeloma proteins which have the desired second specificity of the bispecific monoclonal antibody, or with a hybridoma producing a monoclonal antibody providing the desired second specificity. General techniques for the preparation of cell lines secreting bispecific monoclonal antibodies are known in the art and are, for instance, described in the European patent application 68 763.

After the fusion, cells secreting the desired bispecific antibodies are identified and selected by cultivation in a selective medium as described above and by screening the clones for the ability of a single clone to react with both desired antigens.

The monoclonal antibodies and/or derivatives thereof according to the invention are useful for the qualitative and quantitative determination of IgE, especially in body fluids, e.g. in serum.

For instance, the monoclonal antibodies or derivatives thereof can be used in any of the known

9

immunoassays which rely on the binding interaction between the antigenic determinants of IgE and the paratopes of the monoclonal antibodies. Examples of such assays are radioimmunoassays (RIA), enzyme, immunofluoresence, chemiluminescence, immunoprecipitation, latex agglutination, or hemagglutination immunoassays.

The monoclonal antibodies according to the invention can be used as such or in the form of radioactively labelled derivatives in a radioimmunoassay (RIA). Any of the known modifications of a RIA can be used, for example soluble phase (homogeneous) RIA, solid phase (heterogeneous) RIA, single RIA or double (sandwich) RIA with direct or indirect (competitive) determination of IgE.

An example of such a radioimmunoassay is a sandwich RIA in which a suitable carrier, for example the plastic surface of a microtitre plate or of a test tube, e.g. of polystyrene, polypropylene or polyvinylchloride, glass or plastic beads, filter paper, dextran etc., cellulose acetate or nitrocellulose sheets, magnetic particles or the like, is coated with a monoclonal antibody of the invention by simple adsorption or optionally after activation of the carrier. Then test solutions containing IgE and finally polyclonal antibodies which also react with the antigen and which are radioactively labelled, e.g. with $^{125}$I, are added. The amount of IgE in the test solutions is directly proportional to the amount of bound polyclonal antibodies and is determined by measuring the radioactivity bound to the carrier. The polyclonal antibodies can be replaced by a second radioactively labelled monoclonal antibody of the invention which recognizes a different epitope of the antigen than the first carrier-bound monoclonal antibody.

The monoclonal antibodies according to the invention can be used as such or in the form of enzyme-conjugated derivatives in an enzyme immunoassay. As described above for radioimmunoassays, any of the known modifications of an enzyme immunoassay can be used.

The tests are carried out in an analogous manner to the radioimmunoassays described above using an enzyme label instead of a radioactive label. The amount of immune complex formed which corresponds to the amount of IgE present in the test solutions is determined by adding an enzyme substrate solution. The enzyme substrate reaction results, for example, in a color change which can be observed by eye or with optical measuring devices.

The monoclonal antibodies according to the invention can be used as such or in the form of derivatives conjugated with chemiluminescent markers in a chemiluminescence assay. As described above for radioimmunoassays, any of the known modifications of a chemiluminescence assay can be used.

The tests are carried out in an analogous manner to the radioimmunoassays described above using a chemiluminescent label instead of a radioactive label. The amount of immune complex formed which corresponds to the amount of IgE present in the test solutions is determined by adding a compound triggering luminescence, e.g. $H_2O_2$ and NaOH, and measuring the emission of light with optical measuring devices.

The use according to the invention of monoclonal antibodies and derivatives thereof as described hereinbefore for the determination of IgE also includes other immunoassays known per se, for example immunofluorescence assays, latex agglutination with antibody-coated or antigen-coated latex particles, hemagglutination with antibody-coated or antigen-coated red blood corpuscles, evanescent light assays using an antibody-coated optical fibre and other direct-acting immunosensors which convert the binding event into an electrical or optical signal, or the like.

The monoclonal antibodies and/or derivatives thereof according to the invention are also useful for the determination of IgE-producing cells, preferentially in a plaque forming cell (PFC) assay.

A plaque forming cell assay according to the invention is based on the principles of a solid phase immunoassay. Any of the known modifications of a solid phase immunoassay can be used, for example a radioimmunoassay, an enzyme, immunofluorescence or chemiluminescence immunoassay, or the like.

An example of such a plaque forming cell assay is a PFC assay based on an enzyme-linked immunosorbent assay (ELISA). For determination of the total amount of IgE-producing cells, a suitable carrier as described above for a sandwich RIA is coated with a monoclonal antibody of the invention. A suspension of IgE-producing cells which are obtained from body fluids containing such cells by centrifugation, filtration, or the like, and a second polyclonal or monoclonal antibody specific for IgE, e.g. a monoclonal antibody of the invention recognizing a different epitope of IgE than the first antibody, which is conjugated with an enzyme, e.g. alkaline phosphatase, are added. The amount of IgE-producing cells in the test suspensions is directly proportional to the amount of bound second antibody and is determined by adding an appropriate substrate solution, which results for example in the development of a colored reaction product, and counting the colored spots (plaques). For determination of the fraction of IgE-producing cells which produce IgE directed against a specific allergen, the carrier is first coated with the allergen or an adsorbable conjugate of the allergen before adding a cell suspension as described above. The fraction of IgE in the test suspension which is directed against the allergen binds to the surface-bound allergen and is determined by adding a monoclonal antibody of the invention conjugated with an enzyme and an appropriate substrate solution resulting for example in the development of a coloured reaction product, and counting the colored spots (plaques).

Furthermore, the invention concerns test kits for the qualitative and quantitative determination of IgE and/or IgE producing cells comprising monoclonal antibodies and/or derivatives thereof of the invention and, optionally, other monoclonal or polyclonal antibodies and/or adjuncts.

Test kits according to the invention for a radioimmunoassay contain, for example, a suitable carrier, optionally freeze-dried or concentrated solutions of one or more monoclonal antibodies, solutions of a radioactively labelled monoclonal antibody or of radioactively labelled IgE, standard solutions of IgE, buffer solutions and, optionally, detergents for preventing non-specific adsorption and aggregate formation, pipettes, reaction vessels, calibration curves and the like. One or more of the monoclonal antibodies of the test kit are monoclonal antibodies of the invention. Test kits for the determination of IgE-producing cells which produce IgE directed against a specific allergen additionally contain solutions of the allergen or an adsorbable conjugate of the allergen.

Test kits according to the invention for an enzyme-immunoassay contain, for example, a suitable carrier, optionally freeze-dried or concentrated solutions of one or more monoclonal antibodies, optionally freeze-dried or concentrated solutions of an enzyme-labelled monoclonal antibody, of enzyme-labelled IgE, of a polyclonal anti-IgE serum and/or of enzyme-labelled monoclonal or polyclonal antibodies that recognize and bind the anti-IgE antibody, enzyme substrates in solid or dissolved form, standard solutions of IgE, buffer solutions, detergents, pipettes, reaction vessels, calibration curves, color scale tables and the like. One or more of the monoclonal antibodies of the test kit are monoclonal antibodies of the invention. Test kits for the determination of IgE-producing cells which produce IgE directed against a specific allergen additionally contain solutions of the allergen or an adsorbable conjugate of the allergen.

Moreover, the monoclonal antibodies according to the invention and their derivatives can be used for the qualitative and quantitative determination of surface IgE positive (sIgE$^+$) B cells by any of the known conventional staining techniques, e.g. by flow cytometric analysis. Hitherto, it has been practically impossible to use such techniques for the selective determination of sIgE$^+$ B cells since the reaction of the anti-IgE monoclonal antibodies known in the art with IgE bound on sensitized mast cells and basophils interferes with the staining reaction. The monoclonal antibodies and their derivatives of the invention overcome this severe drawback since they do not recognize IgE on mast cells and basophils.

In addition, the monoclonal antibodies of the invention and/or their derivatives are useful in therapy, in particular for the treatment and/or prophylaxis of allergy.

The therapeutic effect is achieved by downregulating the IgE immune response due to the specific characteristics of the monoclonal antibodies and derivatives thereof according to the invention:

• They are capable of neutralizing formed IgE by binding free IgE and inhibiting the binding of IgE to cells bearing Fc$_\epsilon$ receptors I or II, in particular mast cells and basophils.

• They recognize and bind IgE expressed on the surface of surface IgE positive B cells (sIgE$^+$ B cells) and are therefore useful in depleting the population of such cells which form a "memory pool" resulting in IgE production after a second exposure to the allergen. The possibility to produce monoclonal antibodies, especially chimeric antibodies, of chosen immunoglobulin (sub)classes allows the activation of cellular mechanisms of the host immune system resulting in specific killing of the sIgE$^+$ B cells. This can also be achieved by conjugates of the monoclonal antibodies of the invention with cytotoxic drugs or by bispecific antibodies which will deliver such drugs to the target cells.

• Since the monoclonal antibodies of the invention and their derivatives do not recognize cytophilic IgE on cells bearing Fc$_\epsilon$ receptors 1 or II, e.g. mast cells and basophils, they do not induce mediator release.

• The monoclonal antibodies and derivatives thereof according to the invention also have a long lasting therapeutic effect because they have a significant inhibitory effect on the formation of IgE in the immune response.

In consequence, the monoclonal antibodies of the invention and their derivatives provide a treatment that, rather than treating symptoms, actually affects the underlying cause of allergy, for example by removal of IgE antibodies and surface IgE positive B cells, thus eliminating the potential for an allergic response, and inhibition of IgE formation. It is especially advantageous that the treatment does not require ongoing repeated doses, and that the monoclonal antibodies and their derivatives of the invention can be used for prophylactic treatment by administration prior to detection of any of the symptoms of allergy. Due to their reduced immunogenicity, the chimeric monoclonal antibodies and derivatives thereof according to the invention are especially useful for therapeutic applications and prophylaxis. The therapeutic daily dose for mammals is between approximately 0.1 mg and 10 mg per kg body weight depending on the status of the patient and the mode of application.

The invention also relates to pharmaceutical preparations comprising a monoclonal antibody and/or derivatives thereof according to the invention. The pharmaceutical preparations comprise, for example, the monoclonal antibodies and/or derivatives thereof in a therapeutically effective amount together or in admixture with inorganic or organic, solid or liquid pharmaceutical carriers.

Preferred are pharmaceutical preparations for parenteral application and inhalation. Preparations for intramuscular, subcutaneous or intravenous application or for inhalation are e.g. isotonic aqueous solutions or suspensions, optionally prepared shortly before use from lyophilized or concentrated preparations. The pharmaceutical preparations may be sterilized and contain adjuvants e.g. for conserving, stabilizing, wetting, emulsifying or solubilizing the ingredients, salts for the regulation of the osmotic pressure, buffer and/or compounds regulating the viscosity, e.g. sodium carboxycellulose, dextran, polyvinylpyrrolidone or gelatine. They are prepared by methods known in the art, e.g. by conventional mixing, dissolving or lyophilizing, and contain from approximately 0.01 % to approximately 50 % of active ingredients. The preparations for injections are processed, filled into ampoules or vials, and sealed under aseptic conditions according to methods known in the art.

The following examples illustrate the invention but do not limit it to any extent.

Abbreviations

BPO benzylpenicillin (benzylpenicilloyl)
BSA bovine serum albumin
BSS balanced salt solution
CFA complete Freund's adjuvant
FCS fetal calf serum
HAT hypoxanthine/aminopterin/thymidine
HEPES N-[2-hydroxyethyl]piperazine-N′-[2′-ethanesulfonic acid]
Ig immunoglobulin
i.p. intraperitoneally
i.v. intravenously
MAb monoclonal antibody
PBS phosphate buffered saline
PC phosphorylcholine
rpm revolutions per minute

Example 1: Preparation of hybridoma cell lines secreting rat anti-murine IgE monoclonal antibodies

1.1 Immunization of rats with murine IgE

RA 25 rats (former SIV 50) are immunized with a number of purified murine monoclonal antibodies of immunoglobulin class IgE (IgE MAbs) as listed in Table 1. These IgE MAbs have different specificities to favor induction of anti-isotypic, and not anti-idiotypic, antibodies, i.e. antibodies which selectively recognize the antigenic sites shared by immunoglobulins of the IgE class not present on all other classes of immunoglobulins.

Table 1

| Murine IgE MAbs used for immunization | | |
|---|---|---|
| murine IgE designation | source | specificity |
| aPC4-33 | Ciba-Geigy | phosphorylcholine (PC), group II |
| a-PC7-1-30 (switch mutant) | Ciba-Geigy | PC T$_{15}^{+}$ group I |
| aPC12-3 | Blaser & Heusser, Int. Rev. Immunol. 2, 93, 1987 | PC group II |
| aDNP69-3 | Prof. G. Köhler, Freiburg, Germany | 2,4-dinitrophenol (DNP) |

A mixture of the IgE MAbs of Table 1 (10 μg each) in complete Freund's adjuvant is injected intraperitoneally (i.p.) into the rats. On the 14th day thereafter, a further injection of the same mixture at the same concentration in incomplete Freund's adjuvant is injected i.p.. The serum titres are determined as described in Example 1.3, and the rat with the highest serum titre against murine IgE is then boosted intravenously with the mixture of IgE MAbs of Table 1 in PBS 4 days before the fusion.

1.2 Cell fusion

Cell fusion is accomplished using $10^8$ spleen cells of immunized rats and $3 \times 10^7$ cells from the mouse myeloma cell line Sp2/0-Ag14 (Shulman et al., Nature 176, 269, 1978) in the presence of 1 ml of 50 % polyethylene glycol (PEG 4000, Merck) according to conventional previously described methods (Köhler & Milstein, Nature 256, 495, 1975). After washing, the cells are resuspended in 300 ml of Standard Dulbecco's Minimum Essential Medium (Seromed). 15 % fetal calf serum and $3 \times 10^6$ normal mouse peritioneal exudate cells are added per fusion as feeder cells. The cells are distributed between six 48 x 1 ml Costar plates. The cultures are fed twice weekly with standard HAT selective medium, later with HT medium, for 3 to 6 weeks. When growth of hybridoma cells becomes visible, the culture supernatants are screened for binding to murine IgE by the ELISA test of Example 1.3 and are afterwards tested for histamine release and binding to mouse surface IgE (see Examples 5 and 7).

The selected hybridoma cells can be grown in culture, frozen at -80°C or in liquid nitrogen and then reactivated. The cells are cloned by limiting dilution (Goding, J. Immunol. Methods 39, 285, 1980) and expanded by forming ascites in Balb/c mice primed with pristane (see Example 2.1).

1.3 Selection of hybridomas secreting rat anti-murine IgE by sandwich enzyme-linked immunosorbent assay (ELISA)

The growing hybridomas are tested for the presence of antibodies directed against the murine IgE isotype (anti-murine IgE antibodies) by a sandwich enzyme-linked immunosorbent assay (ELISA).

Plastic microtitre plates are coated with immunoglobulins by incubating 500 μg of the four IgE MAbs of Table 1 (see Example 1.1) or, as controls, of murine MAbs and myeloma proteins (Bionetics) of all other immunoglobulin classes (see Table 2 below) in 50 μl of 0.05 M sodium bicarbonate buffer pH 9.6 for 2 hours at 37°C and 15 hours at 4°C.

EP 0 396 505 A2

Table 2

| Murine MAbs and myeloma proteins of different isotypes employed in the ELISA | | | |
|---|---|---|---|
| H-chain | L-chain | | |
| | $x$ | $\lambda_1$ | $\lambda_2$ |
| $\mu$ | aPC35-2-2 aPC11-1 | MOPC104E | |
| $\gamma 1$ | aPC59-D$_4$ MOPC21 | aPC56-1 | |
| $\gamma 2a$ | aPC55-1-8 UPC10 RPC5 | | |
| $\gamma 2b$ | aPC281-1-1 MOPC195 MOPC141 | | |
| $\gamma 3$ | aPC61-1 J606 | J5606 | |
| $\alpha$ | T15 | | MOPC315 |
| $\epsilon$ | aPC12-3 aPC4-33 aDNP69-3 aTNPSPE7 | | |

After washing with PBS, protein-reactive sites that are still present on the plastic surface are saturated by incubation for 2 hours at 37°C with 150 $\mu$l of PBS-Tween® buffer (0.05 % Tween®20 in PBS containing 0.2 % NaN$_3$ and 1 % BSA, pH 7.4) and the plates are washed with PBS. 100 $\mu$l of cell culture supernatants and corresponding dilutions thereof are incubated for 2 hours at 37°C and, after washing the plates, the bound rat monoclonal antibodies are developed by incubation with 100 $\mu$l of a correspondingly predetermined dilution of a preparation of alkaline phosphatase-labelled goat IgG anti-rat immunoglobulin (Sigma). The amount of enzyme taken up is determined by incubation (30 minutes, 37°C) with 100 $\mu$l of a solution of the enzyme substrate p-nitrophenyl phosphate (1 mg/ml in 10 % diethanolamine buffer containing 0.5 mmol of MgCl$_2$, pH 9.8) and measurement of the optical density of the reacted product at 405 nm (OD$_{405}$) using a Multiscan Photometer (Flow Irvine).

Based on the results of the screening procedure given in Table 3 below, 6 hybridomas designated 18-20, 46-48, 4A1-28, 483-39, 1-5 and 3-11 are selected for further studies. They are derived from 4 different fusions and secrete monoclonal antibodies which exhibit high binding reactivity (100 %) to murine IgE whereas they show only background crossreactivity with MAbs and myeloma proteins of other Ig isotypes. The full designation of hybridoma 1-5 is 654-1-5, but the first three digits are omitted for convenience. The MAbs are designated MAb 18-20, MAb 46-48, MAb 4A1-28, MAb 4B3-39, MAb 1-5 and MAb 3-11, referring to the hybridoma cell line by which they are secreted.

14

Table 3

| Selection of hybridomas for anti-murine IgE isotype specificity | | | | | | | |
|---|---|---|---|---|---|---|---|
| hybridoma | binding (%) to the murine MAbs and myeloma proteins of Table 2 | | | | | | |
| | $\mu$ | $\gamma 1$ | $\gamma 2a$ | $\gamma 2b$ | $\gamma 3$ | $\delta$ | $\epsilon$ |
| 18-20 | 0.05 | <0.002 | 0.009 | 0.006 | 0.013 | 0.006 | 100 |
| 46-48 | <0.3 | <0.3 | <0.3 | <0.3 | 0.54 | <0.3 | 100 |
| 4A1-28 | 0.005 | <0.002 | 0.008 | 0.006 | 0.03 | 0.002 | 100 |
| 4B3-39 | <0.002 | <0.002 | <0.002 | <0.002 | 0.004 | 0.002 | 100 |
| 1-5 | <0.002 | <0.002 | <0.002 | <0.002 | 0.017 | <0.002 | 100 |
| 3-11 | <0.001 | <0.001 | <0.003 | <0.001 | 0.015 | <0.001 | 100 |

Example 2: Production, isolation and purification of the rat anti-murine IgE monoclonal antibodies

2.1 Expansion of hybridomas in vivo and purification of monoclonal antibodies

For ascites production, female Balb/c mice (20-25 g) (Tierfarm Sisseln, Switzerland) are pretreated with 0.3 ml pristane oil (Aldrich) intraperitoneally. 1 to 3 weeks later, the mice receive a second injection of pristane (0.2 ml i.p.) and are simultaneously inoculated i.p. with $2 \times 10^6$ hybridoma cells in 0.2 ml PBS. After 8-10 days, the resulting ascites fluid is collected, centrifugated at 800 x g and stored at -20° C or at -80° C.

Thawed ascites fluid is clarified by centrifugation at 30'000 x g for 1 hour. After removing the top layer containing lipids, the protein concentration is determined and adjusted to 10-12 mg/ml with PBS. The immunoglobulin G fraction (IgG) is precipitated by dropwise addition of 0.9 volumes of saturated ammonium sulfate at 0° C. After 1 hour, the IgG fraction is pelleted by centrifugation for 1 hour at 22'000 x g. The pellet is dissolved in 20 mM Tris-HCl buffer pH 7.9 containing 50 mM NaCl, and is dialyzed against the same buffer overnight at 4° C. The IgG fraction is further purified by anion exchange chromatography on a column of DE52 diethylaminoethyl cellulose (Whatman). The sample is diluted 1:2 (v/v) in 20 mM Tris-HCl pH 7.9 to a final concentration of 25 mM NaCl, and 10 mg of protein per ml of gel are loaded onto the column. The elution is obtained by increasing the sodium chloride concentration from 25 mM to 200 mM (linear gradient). In general, MAbs are eluted around 80 mM NaCl. The fractions are dialyzed against PBS overnight at 4° C and stored at -70° C. Purity is assessed by SDS-PAGE and isoelectric focusing. Puritiy is greater than 95 %.

2.2 Expansion of hybridomas in vitro

Precultures of the hybridoma cell lines are. obtained by culturing hybridoma cells at physiological temperature (around 37° C) in RPMI 1640 medium (Seromed) containing 10 % fetal calf serum (FCS) to a final cell density of $5 \times 10^5$ to $10^6$ cells per ml. The whole preculture is filled into Bellco culture vessels and adjusted to a total volume of 1500 ml with fresh RPMI 1640 medium/10 % FCS. The culture is stirred at around 37° C under 5 % $CO_2$ at 30 rpm for two to three days, then diluted to a total volume of 3000 ml with RPMI 1640/10 % FCS and stirred for another seven to ten days. After this time 95 % of the cells are dead. The culture broth is centrifuged at 1000 x g for 20 min at 4° C. The supernatant is filtered through a filter with pore size 0.2 $\mu$m under sterile conditions. Crude immunoglobulin is precipitated by slow dropwise addition of 0.9 volume equivalents of saturated ammonium sulfate at 0° C. This precipitate is purified as described in Example 2.1.

Example 3: Determination of the isotype of the rat anti-murine IgE monoclonal antibodies

The immunoglobulin class and subclass of the rat anti-murine IgE MAbs of Example 1.3 is determined as described by Ouchterlony. MAb 4B3-39, MAb 1-5 and MAb 3-11 are of class IgG1, MAb 46-48 is of class IgG2a, and MAb 18-20 and MAb 4A 1-28 are of class IgG2b.

Example 4: Analysis of IgE epitopes recognized by the rat anti-murine IgE monoclonal antibodies

Microtitre plates are coated with the rat anti-murine IgE monoclonal antibodies of Example 1.3 in such a manner that approximately 150 ng of monoclonal antibodies are bound per well. 3 $\mu$g (20-fold excess) of the same monoclonal antibody or of a different monoclonal antibody are pre-incubated with 5 ng of $^{125}$I-labelled mouse IgE MAb aPC12-3 in 10 $\mu$l of RIA buffer (PBS containing 1 % BSA and 0.2% NaN$_3$) for 4 hours at 37°C and then incubated in the wells of the coated microtitre plates for 2 hours at 37°C and for 15 hours at 4°C. The plates are then washed and the bound IgE is determined by measuring the radioactivity. The results of this cross-inhibition experiment are given in Table 4.

Table 4

| Cross-inhibition of the rat anti-murine IgE monoclonal antibodies | | | | | | |
|---|---|---|---|---|---|---|
| bound MAb | Inhibition of the binding to IgE by excess monoclonal antibody | | | | | |
| | MAb 4B3-39 | MAb 46-48 | MAb 3-11 | MAb 18-20 | MAb 4A1-28 | MAb 1-5 |
| MAb 4B3-39 | + + + | + + | - | - | - | - |
| MAb 46-48 | + + + | + + + | + + | - | - | - |
| MAb 3-11 | - | + + | + + + | - | - | - |
| MAb 18-20 | - | - | - | + + + | - | - |
| MAb 4A1-28 | - | - | - | - | + + + | + + |
| MAb 1-5 | - | - | - | - | - | + + + |
| - 0-30% inhibition | | | | | | |
| + 31-60% inhibition | | | | | | |
| + + 61-95% inhibition | | | | | | |
| + + + 96-100% inhibition | | | | | | |

Binding of IgE to a surface-bound monoclonal antibody is inhibited completely (more than 95 %) by pre-incubation with the same monoclonal antibody in solution (Table 4, diagonal). The monoclonal antibody MAb 46-48 which cross-reacts with MAb 4B3-39 is likewise cross-inhibited by the monoclonal antibody MAb 3-11, but antibodies MAb 4B3-39 and MAb 3-11 do not have any effect on each other. These three monoclonal antibodies thus recognize partially overlapping, successive epitopes on the IgE molecule. Only one other pair of monoclonal antibodies exhibits any cross-reactivity, namely MAb 4A1-28 and MAb 1-5. The inhibition is, however, only one-way; monoclonal antibody MAb 1-5 inhibits the binding of MAb 4A1-28 to the IgE molecule, but MAb 4A1-28 is unable to inhibit MAb 1-5 from binding to the IgE. These two monoclonal antibodies therefore recognize neighboring epitopes with MAb 1-5 sterically hindering the binding of MAb 4A 1-28 to the IgE molecule. All other monoclonal antibody pairings exhibit no reciprocal effect and therefore bind to spatially separate epitopes on the IgE molecule.

Example 5: Determination of specific histamine release induced by the rat anti-murine IgE MAbs

5.1 Preparation of RBL-2H3 cells

The rat basophilic leukemia clone RBL-2H3 (Europ. num. 11, 317, 1981) is grown in Minimum Essential Medium plus 15 % FCS in 75 cm$^2$ Falcon flasks in a humid incubator at 37°C and with 7.5 % CO$_2$. For the

assay the cells are trypsinized, washed with balanced salt solution and counted. 1 $\mu$g of the murine IgE MAb aPC 12-3 (specific for phosphoryl choline, see Table 1, Example 1.1) is added per $10^5$ RBL-2H3 cells and incubated at 37° C for 30 minutes while rotating. The cells are then washed once in medium, adjusted to $10^5$ cells/50 $\mu$l and incubated for 15 minutes in a shaker water bath at 37° C with

(1) dilutions of the various anti-IgE MAbs (0.5 $\mu$g in 50 $\mu$l),

(2) PC-BSA (1 $\mu$g in 50 $\mu$l) as positive control, or

(3) 50 $\mu$l BSS for determination of spontaneous histamine release, or for 15 minutes at 90° C with

(4) 50 $\mu$l BSS for determination of total histamine release.

The tubes are centrifuged for 5 minutes at 3000 x g and 50 $\mu$l of the supernatant is transferred to another tube for histamine release determination as described in Example 5.2.

## 5.2 Determination of histamine release

60 $\mu$l of SAM cocktail (10 $\mu$Ci$^3$H-adenosyl methionine [Du Pont NEL 155H] in 1 ml PBS + histamine-L-methyl-transferase) are added to the 50 $\mu$l supernatant and incubated for 90 minutes at 37° C in a shaker water bath. 40 $\mu$l of 1.5 N HClO$_4$, then 40 $\mu$l of 10 N NaOH and 500 $\mu$l of isoamyl alcohol/toluol (ratio 2:8) are added to the tube, vortexed for 1 minute and then centrifuged at 190 x g for 5 minutes. The radiolabelled methyl histamine is, under alkaline conditions, in the organic phase. 300 $\mu$l of the organic phase is transferred to vials containing 1 ml ethanol and 10 ml scintillation cocktail (1 litre toluol + 42 ml Liquiflor™, Du Pont) and counted in a Tri-Carb™ liquid scintillation analyser. The measured radioactivity is proportional to the histamine release.

The results are given in Table 5.

Table 5

| Specific histamine release induced by the rat anti-murine IgE MAbs | |
|---|---|
| releasing agent | histamine release (%) |
| MAb 4B3-39 (1 $\mu$g) | 112 |
| MAb 46-48 (1 $\mu$g) | 109 |
| MAb 3-11 (1 $\mu$g) | 109 |
| MAb 18-20 (1 $\mu$g) | 84 |
| MAb 4A1-28 (1 $\mu$g) | 84 |
| MAb 1-5 (1 $\mu$g) | < 0.5 |
| MAb 1-5 (2 $\mu$g) | < 0.5 |
| PC-BSA (1 $\mu$g) | 100* |

* 2.14 ng histamine/$10^5$ cells

The results clearly indicate that various anti-IgE MAbs induce histamine release from IgE sensitized RBL-mast cells. However, MAb 1-5 does not induce histamine release although it is highly reactive with non-mast cell bound IgE.

## Example 6: Inhibition of IgE binding to Fc$_\epsilon$ receptor I bearing mast cells by the rat anti-murine IgE monoclonal antibodies

The capability of the rat anti-murine IgE monoclonal antibodies of Example 1.3 to inhibit the binding of $^{125}$I-labelled murine IgE to PB3 mast cells (Ball et al., Differentiation 24, 74,1983) is determined in an inhibition immunoassay as described in the following.

Flat-bottomed polyvinyl chloride microtitre plates are coated with 50 $\mu$l of poly-L-lysine at a concentration of 20 $\mu$g/ml in PBS. After washing with PBS 3 x $10^5$ PB3 mast cells (50 $\mu$l at 6 x $10^6$/ml) are added to

EP 0 396 505 A2

each well and then gently immersed in freshly prepared 0.25 % glutaraldehyde in PBS at 4°C and left for 5 minutes. With a gentle flicking motion the glutaraldehyde is removed from the wells and the plates are then washed in three successive 1 litre PBS baths. Any remaining protein-reactive sites that are still present on the plastic surface are saturated by incubating the plates for 1 hour at 37°C with 225 $\mu$l of PBS containing 1 % BSA and 0.2 % NaN$_3$. After washing in PBS, 25 $\mu$l of buffer or 25 $\mu$l of dilutions of the anti-IgE monoclonal antibodies in buffer are added to the appropriate wells. 25 $\mu$l of $^{125}$I-labelled mouse IgE MAb aPC12-3 (40'000 cpm, ca. 3 ng) are added to each well. The plates are gently shaken and incubated for 15 minutes at 37°C and 1 hour at room temperature. 175 $\mu$l of PBS are then added to each well and the plates are inversed over plastic-backed tissues until the wells are empty. The plates are then washed 4 times in 1 litre PBS baths and dried. The radioactivity in the wells is counted and the degree of inhibition is calculated. The results for antibody MAb 1-5 are shown in Table 6.

Table 6

| Inhibition of $^{125}$I-IgE binding to PB3 mast cells by the anti-murine IgE MAb 1-5 | |
|---|---|
| Amount of MAb 1-5 (ng/well) | Degree of inhibition (%) |
| 1000 | 73.3 |
| 100 | 66.0 |
| 10 | 52.0 |
| 1 | 9.5 |
| * 50 % inhibition = 9.5 ng MAb 1-5 | |

Example 7: Binding of the rat anti-murine IgE MAbs to IgE bearing B cells and to IgE coated mast cells

The binding of the rat anti-murine IgE MAbs to IgE bearing B cells and to IgE coated mast cells is determined by flow cytometric analysis.

Hybridoma cells producing the IgE MAb aPC12-3 (see Table 1) serve as surface IgE bearing B cells (sIgE+ B cells). Cells of the rat basophilic leukemia cell line RBL-2H3 sensitized with the IgE MAb aPC12-3 as described in Example 5.1 are used as IgE coated mast cells.

Staining is performed as follows: 1-5 $\mu$g of biotinylated rat anti-murine IgE MAbs are incubated with 10$^6$ cells in 200 $\mu$l RIA buffer containing 5 % FCS for 30 minutes at 4°C. After washing, the cells are resuspended in 200 $\mu$l RIA buffer containing 3 $\mu$l avidin-FITC (Becton-Dickinson, No. 9011) for 30 minutes at 4°C and washed again. They are resuspended in 200 $\mu$l RIA buffer and analyzed by flow cytometry (Ortho Diagnositc System 50 HH connected with a 2150 computer).

18

EP 0 396 505 A2

Table 7

| Binding of the rat anti-murine IgE MAbs to sIgE[+] cells and to IgE coated mast cells | | |
|---|---|---|
| anti-murine IgE MAb (1 $\mu$g) | % stained cells | |
| | sIgE[+] B cells | IgE coated RBL-2H3 cells |
| 0 | < 1 | < 1 |
| MAb 4B3-39 | 99.9 | 99.9 |
| MAb 46-48 | 99.3 | 99.8 |
| MAb 3-11 | 99.9 | 99.9 |
| MAb 4A1-28 | 99.4 | 99.7 |
| MAb 1-5 | 99.8 | < 1.2 |
| non-specific rat MAb | < 1 | < 1 |

The results clearly demonstrate that various anti-murine IgE MAbs react with both types of cells. However, MAb 1-5 does not bind to the IgE coated mast cells although it is highly reactive with surface IgE on the B cells.

Example 8: In vitro effect of the rat anti-murine IgE MAbs

8.1 Antigen induced in vitro immune response assay

In a first test series, the in vitro immune response induced by a specific antigen is studied.

8.1.1 Induction of benzylpenicillin (BPO)-specific antibodies

Balb/c mice (Sisseln animal farm) are primed twice i.p. two weeks apart, with 10 $\mu$g of benzylpenicillin (BPO, Serva) coupled to keyhole limpet haemocyanin (KLH, Calbiochem) in 200 $\mu$l Alu-S-gelTM (Serva). The coupling is carried out according to Nakawaga et al. (Int. Archs. Allergy Appl. Immunol. 63,212,1980). No earlier than three months after the second priming injection the spleens are removed from three of the mice, homogenized and washed 3 times in cold BSS. The cells are added to 24-well plates at a concentration of $10^6$ ml in Iscove's medium (Seromed FO465) with 5 % FCS (screened for in vitro culture) in the presence of 0.5 $\mu$g BPO-KLH with different concentrations of the various rat antimurine IgE MAbs. The plates are incubated in a humid incubator at 37°C and 7.5 % $CO_2$ for 6 days. Supernatants are then transferred to another plate for determination of IgG1 and IgE in the ELISA described in Examples 8.1.3 and 8.1.4. The cells are tested for their activity of antibody production in the plaque forming cell assay of Example 8.1.2.

8.1.2 Plaque forming cell assay for determination of antibody producing cells

A new ELISA plaque assay for the detection of antibody producing cells is developed in order to analyze the effect of anti-IgE monoclonal antibodies on the development of such cells.

1 ml of cold BSS is added to the wells of the microtitre plates of Example 8.1.1 and the cells are carefully resuspended and transferred to 5 ml Falcon tubes. The volume is adjusted to 3 ml by the addition of more cold BSS and the tubes are kept on ice.

48-well plates are coated with BPO coupled to BSA (coupling according to the procedure described in Example 8.1.1 for KLH; 200 $\mu$l at 500 $\mu$g/ml) in PBS for 15 hours at 4°C. After washing the plates are incubated for 1 hour at 37°C with 1 ml of PBS containing 1 % BSA, 0.2 % $NaN_3$ and 0.05 % Tween® 20 to block any protein-reactive sites that are still free. The plates are again washed in PBS. The harvested cells

19

are centrifuged for 7 minutes at 190 x g and resuspended in 1 ml of culture medium. Depending on the isotype being measured, 1 ml of different dilutions of cells (1/1 for IgE, 1/10 for IgG2 and 1/30 for IgG1) are added to the coated plates which are then incubated at 37°C in a humid incubator with 7.5 % $CO_2$ for 18 hours. After washing, 100 $\mu l$ of alkaline phosphatase-labelled antibodies with the appropriate isotype specificity are added to the plates and incubated for 2 hours at 37°C. After washing with PBS, 100 $\mu l$ of the substrate 5-bromo-4-chloro-3-indolylphosphate (BCIP, Sigma) in 2-amino-2-methyl-1-propanol buffer (AMP, Fluka) is added to the wells and incubated at room temperature until distinct blue-colored spots (plaques) become visible (approximately 60 minutes).

The results are given in Table 8 (see Example 8.2.3).

### 8.1.3 ELISA for determination of BPO-specific IgE antibodies in the cell supernatants

BPO-specific IgE antibodies in the cell supernatants of Example 8.1.1 are measured quantitatively by the ELISA described in the following.

1 $\mu g$ of BPO-BSA in 50 $\mu l$ of 0.05 M sodium bicarbonate buffer (pH 9.6) are incubated for 2 hours at 37°C or for 15 hours at 4°C in plastic microtitre plates. After washing with PBS, protein-reactive sites that are still present on the plastic surface are saturated by incubating for 2 hours at 37°C with 200 $\mu l$ of PBS-Tween® buffer (0.05 % Tween® 20 in PBS containing 0.2 % $NaN_3$, pH 7.4), and the plates are washed with PBS. 50 $\mu l$ of cell supernatant and dilutions thereof are incubated for 2 hours at 37°C or for 15 hours at 4°C. 50 $\mu l$ of dilutions of known concentration of the BPO-specific mouse IgE MAb 13-10 (Ciba-Geigy) are also incubated for 2 hours at 37°C and 15 hours at 4°C. After washing the plates, the bound IgE-specific antibodies are developed by incubation with 50 $\mu l$ of a correspondingly predetermined dilution of a preparation of biotin-conjugated monoclonal rat anti-murine IgE for 2 hours at 37°C, washing the plates again, and incubating with 50 $\mu l$ of alkaline phosphatase-labelled avidin (Calbiochem) at 1/1000 for a further 1 hour at 37°C. The amount of enzyme taken up is determined by incubation (1 hour, room temperature) with 150 $\mu l$ of the enzyme substrate p-nitrophenyl phosphate (1 mg/ml in 10 % diethanol-amine buffer containing 0.5 mmol of $MgCl_2$, pH 9.8) and measurement of the optical density of the reacted product at 405 nm ($OD_{405}$) using a Multiskan photometer (Flow Irvine, Scotland). The concentration of BPO-specific IgE antibodies in the cell supernatants is calculated by using the standard curve obtained from the BPO-specific IgE monoclonal antibody dilutions.

### 8.1.4 ELISA for determination of BPO-specific IgG1 antibodies in the cell supernatants

BPO-specific IgG1 antibodies in the cell supernatants of Example 8.1.1 are measured quantitatively in an ELISA which is analogous to the ELISA of Example 8.1.3.

A standard curve is obtained by incubation of 50 $\mu l$ of dilutions of known concentrations of the BPO-specific mouse IgG1 MAb kl 16 (obtained from Dr. K. Blaser, Swiss Inst. for Allergy and Asthma Research, Davos). The bound BPO-specific IgG1 antibodies are developed by incubation with 50 $\mu l$ of a corresponding predetermined dilution of a preparation of alkaline phosphate-labelled rabbit IgG anti-mouse IgG1. The amount of enzyme taken up is determined in the same way as for IgE, and the concentration of BPO-specific IgG1 antibodies is calculated by using the standard curves obtained from the BPO-specific monoclonal antibody dilutions.

### 8.2 Polyclonally induced in vitro immune response assay

In a second test series, the polyclonally induced in vitro immune response is studied.

### 8.2.1 Polyclonal induction of antibodies of diverse specificities

Percoll (Pharmacia) isolated small resting B cell cultures (Julius et al., Eur. J. Immunol. 14 753,1984) are setup in RPMI 1640 medium (Gibco) supplemented with L-glutamine (2 mM), penicillin/streptomycin (100 IU/ml), 2-mercaptoethanol (2 x 10⁵ M), sodium pyruvate (1 mM) and 10 % selected fetal calf serum (Seromed) in 5 % $CO_2$ at 37°C. 5 x 10⁵ cells/ml are cultured for 7 days in the presence or absence of mitogen (lipopolysaccharide [LPS] from E. coli B 026:B6 [Difco 3920-10]; 25 $\mu g/ml$) and murine interleukin

EP 0 396 505 A2

4, and different concentrations of the rat anti-murine IgE MAbs in a total volume of 0.2 ml in 96-well flat bottom microtitre plates (Costar). Supernatants of the cell cultures are harvested and analyzed for total IgE and IgG3 in the sandwich ELISA described in Examples 8.2.2 and 8.2.3.

8.2.2 ELISA for determination of the total amount of IgE in the cell supernatants

The total amount of IgE antibodies in the cell supernatants of Example 8.2.1 is measured by the ELISA described in the following.

1 $\mu$g of the rat anti-murine IgE MAb 4B3-39 in 50 $\mu$l of 0.05 sodium bicarbonate buffer (pH 9.6) is incubated for 2 hours at 37°C and for 15 hours at 4°C in plastic microtitre plates. After washing with PBS, protein reactive sites that are still present on the plastic surface are saturated by incubating for 2 hours at 37°C with 200 $\mu$l of PBS-Tween® buffer (0.05 % Tween® 20 in PBS containing 0.2 % $NaN_3$, pH 7.4). The plates are washed with PBS, and 50 $\mu$l of cell supernatant and dilutions thereof are incubated for 2 hours at 37°C or for 15 hours at 4°C. 50 $\mu$l of dilutions of known concentrations of a monoclonal mouse IgE antibody are also incubated for 2 hours at 37°C or for 15 hours at 4°C.

After washing the plates, the bound IgE antibodies are developed as described for BPO specific IgE antibodies in Example 8.1.3 using the rat anti-murine IgE MAb 3-11. The total amount of IgE antibodies in the cell supernatants is calculated by using the standard curve obtained from the IgE monoclonal antibody dilutions.

The results are given in Table 8 (see Example 8.2.3).

8.2.3 ELISA for determination of the total amount of IgG3 in the cell supernatants

The total amount of IgG3 antibodies in the cell supernatants of Example 8.2.1 is measured in an ELISA which is analogous to the ELISA of Example 8.2.2.

As coating antibody, a rabbit IgG anti-mouse IgG3 (Ciba-Geigy) is used. A standard curve is obtained by incubation of dilutions of known concentrations of the monoclonal mouse IgG3 antibody aPC61-1 (see Table 2). The biotin-conjugated rat monoclonal anti-mouse kappa antibody Ra 33-18-12 (obtained from Prof. G. Hämming, Freiburg, Germany) is used as developing antibody.

The results are shown in Table 8 below.

Table 8

| Inhibition of in vitro IgE response by rat anti-murine IgE MAbs | | | | |
|---|---|---|---|---|
| MAb | % inhibition of immunoglobulin response | | | |
| | antigen induced[1] | | polyclonally induced[2] | |
| | anti-BPO IgGl | anti-BPO IgE | total IgG3 | total IgE |
| MAb 1-5 | 0 | 65 | 0 | 55 |
| MAb 4B3-39 | 5 | 100 | 0 | 88 |

(1) 6 day cultures of mouse spleen cells ($10^6$/ml) in the presence of 0.5 $\mu$g/ml BPO-KLH and 1 $\mu$g/ml anti-IgE MAbs; analysis by plaque forming cell assay (Examples 8.1.3, 8.1.4); 100% = 6640 ± 1527 IgGl plaque forming ells (PFC) or 53 ± 11 IgE PFC
(2) 7 day cultures of Percoll™ isolated small resting B cells (5 x $10^5$/ml) in the presence of LPS (25 $\mu$g/ml) and 1 $\mu$g/ml anti-IgE MAbs; analysis by ELISA (Examples 8.2.2, 8.2.3); 100 % = 479 ± 200 $\mu$g/ml IgG3 or 518 ± 64 $\mu$g/ml IgE

21

## Example 9: In vivo effect of the rat anti-murine IgE MAbs

### 9.1 Induction and determination of BPO-specific antibodies

6-8 week old Balb/c mice (Sisseln animal farm) are immunized i.p. twice (two weeks apart) with 0.2 ml of 2 % aqueous aluminium hydroxide (Serva) containing 10 µg BPO-KLH (see Example 8.1.1). One year later these pre-immunized mice are treated as follows:

Group 1(6 mice) receives 0.2 ml i.p. of Al(OH)₃ containing 50 µg of BPO-KLH on day 0;

group 2(6 mice) receives 200 µg of purified rat anti-murine IgE MAb 1-5 on days - 1 (i.p.), 0 (iv.), 2 (i.p.) and 5 (i.v.); and

groups 3 & 4 (both 6 mice) are treated identically to group 2 but with rat anti-murine IgE MAb 4B3-39 and with normal rat IgG (Calbiochem), respectively.

In addition, groups 2,3 and 4 receive 0.2 ml i.p. of Al(OH)₃ containing 50 µg of BPO-KLH on day 0.

On day 14, all the mice are bled and the serum is analyzed for BPO-specific IgE and IgG1 by the ELISA tests described in Examples 8.1.3 and 8.1.4 for cell supernatants. The results are shown in Table 9 (see Example 9.2).

### 9.2 Plaque forming cell assay for determination of antibody producing cells

Mice are pre-immunized as described in Example 9.1. One year later, the mice are treated identically as in Example 9.1 with the exception that groups 2,3 and 4 receive antibody on days -1, 0, 1 and 3.

On day 6, the spleens are removed from the mice and analyzed by the plaque forming cell assay described in Example 8.1.2. The results are shown in Table 9 below.

Table 9

| In vivo effect of the rat anti-murine IgE MAbs | | | group 1 (control) | group 2 (MAb 1-5) | group 3 (MAb 4B3-39) | group 4 (normal rat IgG) |
|---|---|---|---|---|---|---|
| Serum | IgGl mg/ml | | 1.25 ± 0.26 | 1.46 ± 0.11 | 1.39 ± 0.22 | 1.58 ± 0.15 |
| level (day 14) | IgE µg/ml | | 47.66 ± 10.2 | 7.81 ± 5.1 | 3.52 ± 2.0 | 42.97 ± 9.4 |
| PFC per | IgGl | | 297 ± 59 | 271 ± 55 | 266 ± 87 | 577 ± 103 |
| 10⁶ cells | IgE | | 23 ± 2 | 8 ± 2 | 14 ± 2 | 28 ± 5 |
| (1) mean of 6 animals ± 1 SEM (standard error of means) | | | | | | |
| (2) mean of 5 animals ± 1 SEM (standard error of means) | | | | | | |

## Example 10: Preparation of hybridoma cell lines secreting mouse anti-human IgE monoclonal antibodies

### 10.1 Immunization of mice with human IgE

Biozzi mice (Inst. Curie, Paris) are immunized with purified human antibodies of immunoglobulin class IgE as listed in Table 10. These IgE antibodies have different specificities to favor induction of anti-isotypic antibodies.

Table 10

| Human IgE used for immunization | | |
|---|---|---|
| human IgE designation | source | characteristics |
| PS | Prof. K. Jshizaka, Johns Hopkins Univ., Baltimore, USA | produced by a human IgE myeloma |
| WT | Prof. D. Standworth, Univ. of Birmingham, England | produced by a human IgE myeloma |
| JW8 | Serotec (see Neuberger et al., Nature 314, 268, 1985) | chimeric Mab with human ε constant regions and murine variable regions specific for NIP |

A mixture of the IgE antibodies of Table 10 (25 μg each) in complete Freund's adjuvant is injected intraperitoneally (i.p.) into the mice. On the 14th day thereafter, a further injection of the same mixture at the same concentration in incomplete Freund's adjuvant is injected i.p.. The serum titres are determined as described in Example 1.3, and the mouse with the highest serum titre against human IgE is then boosted half intravenously, half intraperitoneally with the mixture of IgE antibodies (7.5 μg of each) of Table 10 in PBS 3 days before the fusion.

### 10.2 Cell fusion

Cell fusion is accomplished using $10^8$ spleen cells of an immunized mouse and $3 \times 10^7$ cells from the mouse myeloma cell line PAI (J.W.Stocker, H.K. Forster, V. Miggiano, C. Stähli, G. Staiger, B. Takacs and Th. Staehelin, Generation of two new mouse myeloma cell lines "PAI" and "PAI-O" for hybridoma production, Research Disclosure 21713, May 1982, p. 155) in the presence of 1 ml of 50 % polyethylene glycol as described in Example 1.2. The culture supernatants are screened for binding to human IgE by the ELISA test and the RIA of Example 10.3. Afterwards they are tested for histamine release according to Example 11.

The selected hybridoma cells can be grown in culture, frozen at -80° C or in liquid nitrogen and then reactivated. The cells are cloned by limiting dilution (Goding, J. Immunol. Methods 39,285,1980) and expanded by forming ascites in nu/nu or Balb/c mice primed with pristane (see Example 10.4).

### 10.3 Selection of hybridomas secreting mouse anti-human IgE

### 10.3.1 Sandwich enzyme-linked immunosorbent assay (ELISA)

The growing hybridomas are tested for the presence of antibodies directed against the human IgE isotype (anti-human IgE antibodies) by a sandwich enzyme-linked immunosorbent assay (ELISA) in analogy to the ELISA of Example 1.3.

Plastic microtitre plates are coated with immunoglobulins by incubating 1 μg of the IgE antibodies of Table 10 (see Example 10.1) or, as controls, of human myeloma proteins (WHO Standards) of all other immunoglobulin classes (see Table 11 below) in 50 μl of 0.05 M sodium bicarbonate buffer pH 9.6. Incubations and whasings are done as described in Example 1.3. The bound mouse monoclonal antibodies are developed with alkaline phosphatase-labelled monoclonal rat IgG anti-mouse x light chain Ra 33-18-12.

### 10.3.2 Solid-phase radioimmunoassay (RIA)

In a second screen, the hybridomas of Example 10.2 are tested for antibodies directed against the human IgE isotype (anti-human IgE antibodies) by a solid-phase radioimmunoassay (RIA).

Polyvinyl chloride microtitre plates are coated by incubating 1 $\mu$g of sheep anti-mouse Ig antibodies in 50 $\mu$l of 0.05 M sodium bicarbonate buffer pH 9.6 for 2 hours at 37°C and 15 hours at 4°C. After washing with PBS, any protein-reactive sites that are still present on the plastic surface are saturated by incubation for 2 hours at 37°C with 200 $\mu$l of PBS-Tween® buffer (0.05 % Tween® 20 in PBS containing 0.2 % NaN$_3$ and 1 % BSA, pH 7.4), and the plates are washed with PBS. 50 $\mu$l of all culture supernatants are incubated in the plates for 4 hours at 37°C and, after washing the plates with PBS, 50 $\mu$l of $^{125}$I-iodinated human IgE is added and the plates are incubated for 1 hour at 37°C. The plates are washed and the amount of IgE bound is determined by counting the wells in a Packard CobraTM 5005 gamma-counter.

Table 11

| Purified human myeloma proteins of different isotypes employed in the ELISA and RIA | | | |
|---|---|---|---|
| H-chain | L-chain | | |
| | x | λ | chimeric |
| μ | Jakop | Orelli | |
| γ1 | HU 0781 | Lob 0284 | |
| γ2 | G263 0783 | Ma 0981 | |
| γ3 | Val 1080 | Hun 1283 | |
| γ4 | Bru 1183 | Gta 0184 | |
| α | GRA 0386 | SCHZ 0386 | |
| | SCHN 0386 | | |
| ε | WT | PS | JW8 |

Based on the results of the screening procedure 7 hybridomas designated F1-18-22, F1-30-3, F1-43-17, F1-62-48, F1-73-63, F1-74-20 and F1-75-14 are selected for further analysis. They secrete monoclonal antibodies which exhibit binding reactivity to human IgE whereas they show only background crossreactivity with myeloma proteins of other Ig isotypes. The anti-IgE MAbs are designated according to the hybridoma cell line by which they are secreted.

10.4 Production, isolation and purification of the mouse anti-human IgE monoclonal antibodies

Hybridomas are expanded in vivo in nu/nu mice or in Balb/c mice inoculated with $10^7$ hybridoma cells as described in Example 2.1. For expansion in vitro, the procedure of Example 2.2 is followed except for the cell culture medium which is DMEM (Gibco) in place of RPMI 1640. The immunoglobulin G fraction is precipitated and chromatographed as described in Example 2.1.

10.5 Determination of the isotype of the mouse anti-human IgE monoclonal antibodies

The immunoglobulin class and subclass of the mouse anti-human IgE MAbs of Example 10.3 is determined as described by Ouchterlony. All monoclonal antibodies tested are of class IgG1.

Example 11: Determination of specific histamine release induced by the mouse anti-human IgE MAbs

The mouse anti-human IgE MAbs are tested for their ability to induce histamine release by a method according to Subramanian & Bray (J. Immunol. 138,271,1987) described in the following.

## 11.1 Preparation of mast cells

Human peripheral blood from healthy volunteers is collected in siliconized tubes and mixed with 6 % dextran (2 ml for 8 ml blood) in EDTA-coated plastic tubes. After mixing by inversion, the cells are allowed to sediment at room temperature, and the leukocyte-rich plasma layer is drawn off and centrifuged at 1'000 x g for 10 minutes. The supernatant is aspirated and the cell pellet is suspended in HEPES buffer solution (25 mM HEPES, 0.9 % NaCl, 0.5 mM glucose, pH 7.4) and recentrifuged at 1'000 x g for 10 minutes. The washing step is repeated twice. The final cell pellet is resuspended in the same buffer containing 0.6 mM $CaCl_2$ and 0.6 mM $MgCl_2$.

## 11.2 Determination of histamine release

Aliquots of approximately 1000 cells are challenged with an appropriate dose of anti-human IgE MAbs of Example 10.3 (1/3 dilution) for 15 minutes at 37°C. Total histamine content of cells is estimated from supernatants of lysed cells (95°C, 10 minutes). Other test compounds are added at the time of challenge. All tests are run in triplicate, and duplicate histamine estimates are performed for each sample.

After incubation, aliquots of test supernatants are assayed for histamine content by using an enzymatic-isotopic assay. 50 $\mu$l of supernatant (or an aliquot diluted to 50 $\mu$l with phosphate buffer, pH 7.9) are incubated for 90 minutes at 37°C with 60 $\mu$l of a cocktail containing rat kidney histamine methyltransferase (25-30 $\mu$g protein) and 0.6 $\mu$Ci [$^3$H]-S-adenosyl methionine (Amersham, 60-80 Ci/mmol). The reaction is terminated by addition of 40 $\mu$l of 1.5 N $HClO_4$ and mixing on a microfuge rack vibrater (Eppendorf). Subsequently, 40 $\mu$l of 10N NaOH are added, followed after mixing by 500 $\mu$l of toluene:isoamyl alcohol mixture (80:20 v/v). The aliquots are mixed thoroughly to extract the labelled methyl histamine formed by the enzymatic reaction. The tubes are then centrifuged at 4'000 rpm for 5 minutes, and 300 $\mu$l of the organic phase is transferred to polypropylene scintillation vials. After addition of 1 ml of ethanol and 10 ml of scintillation cocktail (LiquiforTM; diluted with scintillation grade toluene to give a final concentration of 4 g/l PPO and 50 mg/l POPOP). The radioactivity is counted in a liquid scintillation counter (TricarbTM 4530; Packard).

Out of the 7 tested anti-human IgE MAbs, MAb F1-43-17 does not induce histamine release (less than 5 %) up to 10 $\mu$g/ml MAb from mast cells which are coated with IgE in vivo, although it expresses high reactivity to human IgE as determined in the ELISA of Example 10.3.1. The results are collected in Table 12.

Table 12

| Anti-human IgE activity and histamine release of mouse anti-human IgE MAbs | | |
|---|---|---|
| MAb | anti-human IgE activity A [1] ($\mu$g/ml) | histamine release activity B [2] ($\mu$g/ml) | ratio A / B |
| F1-18-22 | 1.83 ± 0.65 [3] | 0.13 ± 0.02 | 14.1 |
| F1-30-3 | 7.54 ± 3.64 | 0.31 ± 0.19 | 24.3 |
| F1-43-17 | 1.84 ± 0.77 | > 10 | < 0.1 |
| F1-62-48 | 5.65 ± 3.0 | 0.85 ± 0.15 | 6.6 |
| F1-73-63 | 4.78 ± 3.31 | 0.90 ± 0.3 | 5.3 |
| F1-74-20 | 4.66 ± 2.57 | 1.33 ± 0.18 | 3.5 |
| F1-75-14 | 9.72 ± 5.76 | 0.90 ± 0.20 | 10.8 |

(1) determined in the sandwich ELISA assay according to Example 10.3.1; concentration which results in an optical density reading of 0.5.
(2) determined according to Example 11.2; concentration which results in 5 % of the maximal release.
(3) Standard deviation

## 11.3 Determination of inhibition of IgE binding to Fc$_\epsilon$ receptor II bearing lymphoblastoid cells

Monoclonal anti-human IgE antibodies are tested for their ability to inhibit the binding of IgE-coated latex particles to Fc$_\epsilon$ receptor II bearing cells as described in the following.

Latex beads (Sigma) are coated with a conjugate of 4-hydroxy-3-iodo-5-nitrophenylacetic acid (NIP) with BSA (Brownstone et al., Inmunol. 10,465,1966) according to the manufacturer's specifications. The beads are washed, resuspended in PBS containing 0.1 % BSA, 0.2 % NaN$_3$, pH 7.4, and incubated with 100 $\mu$g human IgE chimeric anti-NIP antibody JW8 (Neuberger et al., Nature 314,268,1985) in 500 $\mu$l for 45 minutes at room temperature, followed by washing. 100 $\mu$g of anti-human IgE monoclonal antibodies at a concentration of 0.1 mg/ml (or buffer as control) are incubated with 20 $\mu$l of appropriately diluted IgE-coated latex bead suspension for 45 minutes at room temperature, followed by the addition of 2 x 10$^5$ lymphoblastoid RPMI 8866 cells (Jensen et al., Immunol. 53, 1, 1984) in 100 $\mu$l buffer and incubation for another 60 minutes at room temperature. The inhibition of latex agglutination to RPMI 8866 cells is determined by microscopical inspection.

Indeed, anti-human IgE monoclonal antibodies are found which inhibit the interaction between IgE-coated particles and the RPMI 8866 cells.

## Claims

1. A monoclonal antibody directed against isotypic determinants of immunoglobulin E (IgE) which inhibits the binding of free IgE to cells bearing Fc$_\epsilon$ receptors I or II and recognizes IgE expressed on the surface of IgE surface positive B cells, but does not recognize IgE bound to the surface of cells bearing Fc$_\epsilon$ receptors I or II and does not trigger mediator release from such cells, and a derivative thereof retaining the specificity of the antibody from which it is derived.

2. A monoclonal antibody as claimed in claim 1 which does not recognize IgE bound on the surface of Fc$_\epsilon$ receptor 1 bearing mast cells and basophils and does not trigger anaphylactic mediator release from such cells, and derivatives thereof.

3. A monoclonal antibody as claimed in claim 1 which specifically inhibits IgE formation in the immune response, and a derivative thereof.

4. A monoclonal antibody as claimed in claim 1 which is of immunoglobulin class IgG, and a derivative thereof.

5. A monoclonal antibody as claimed in claim 1 directed against isotypic determinants of murine IgE, and a derivative thereof.

6. A monoclonal antibody as claimed in claim 5 which is a rat antibody, and a derivative thereof.

7. The monoclonal antibody as claimed in claim 6 with the designation MAb 1-5, and a derivative thereof.

8. A monoclonal antibody as claimed in claim 1 directed against isotypic determinants of human IgE, and a derivative thereof.

9. A monoclonal antibody as claimed in claim 8 which is a mouse or rat antibody, and a derivative thereof.

10. A monoclonal antibody as claimed in claim 8 which is a mouse antibody, and a derivative thereof.

11. The monoclonal antibody as claimed in claim 10 with the designation MAb F1-43-17, and a derivative thereof.

12. A monoclonal antibody as claimed in claim 8 which is a chimeric animal/human antibody, and a derivative thereof.

13. A chimeric monoclonal antibody as claimed in claim 12 which consists of murine variable regions and human constant regions, and a derivative thereof.

14. A bispecific monoclonal antibody comprising two different antigen binding portions, one derived from a monoclonal antibody as claimed in claim 8 and the second derived from an antibody with a different specificity, and a derivative thereof.

15. A bispecific monoclonal antibody as claimed in claim 14 in which the second antigen binding portion is derived from an antibody directed against a cytostatic or cytotoxic substance, and a derivative thereof.

16. A derivative of a monoclonal antibody as claimed in claim 1 which is an antibody fragment.

17. A derivative of a monoclonal antibody as claimed in claim 1 which is an antibody conjugate with an enzyme, a fluorescent marker, a chemiluminescent marker, a metal chelate, avidin, biotin, or the like.

18. A derivative of a monoclonal antibody as claimed in claim 1 which is a radioactively labelled antibody.

19. A process for the preparation of monoclonal antibodies and derivatives thereof as claimed in claim 1 wherein cells of a continuous cell line secreting said monoclonal antibodies are multiplied in vitro or in vivo and, when required, the obtained monoclonal antibodies are isolated and/or converted into derivatives thereof.

20. A continuous cell line which is a hybridoma cell line secreting monoclonal antibodies as claimed in claim 1.

21. A hybridoma cell line as claimed in claim 20 which is a hybrid of myeloma cells and B lymphocytes of a mammal immunized with IgE.

22. A hybridoma cell line as claimed in claim 20 which is a hybrid of mouse myeloma cells and B lymphocytes of a rat immunized with murine IgE.

23. The hybridoma cell line as claimed in claim 22 with the designation 654-1-5 which was deposited at the European Collection of Animal Cell Cultures under the number ECACC 89041902 on April 19,1989.

24. A hybridoma cell line as claimed in claim 20 which is a hybrid of mouse myeloma cells and B lymphocytes of a syngeneic mouse immunized with human IgE.

25. The hybridoma cell line as claimed in claim 24 with the designation F1-43-17 which was deposited at the European Collection of Animal Cell Cultures under the number ECACC 90032210 on March 22,1990.

26. A process for the preparation of hybridoma cell lines as claimed in claim 20 wherein a suitable mammal is immunized with IgE, antibody-producing cells of this mammal are fused with cells of a continuous cell line, the hybrid cells obtained in the fusion are cloned, and cell clones secreting the desired monoclonal antibodies are selected.

27. A continuous cell line which is a transfectoma cell line secreting chimeric animal/human monoclonal antibodies as claimed in claim 12.

28. A process for the preparation of transfectoma cell lines as claimed in claim 27 wherein cells of a continuous cell line are transfected with nucleic acid constructs encoding the chimeric animal/human light and/or heavy chains of the desired chimeric monoclonal antibodies, the transfected cells obtained are cloned, and cell clones secreting the desired chimeric monoclonal antibodies are selected.

29. A continuous cell line secreting bispecific monoclonal antibodies as claimed in claim 14.

30. A process for the preparation of a continuous cell line as claimed in claim 29 wherein cells producing heavy and/or light chains of a monoclonal antibody directed against isotypic determinants of human IgE and cells producing heavy and/or light chains of a monoclonal antibody with a different specificity are fused, the hybrid cells obtained in the fusion are cloned, and cell clones secreting the desired bispecific monoclonal antibodies are selected.

31. The use of a monoclonal antibody and/or derivative thereof as claimed in claim 1 for the qualitiative and quantitative determination of IgE.

32. The use as claimed in claim 31 of a monoclonal antibody and/or derivative thereof in a radioimmunoassay, an enzyme immunoassay or a chemiluminescence immunoassay.

33. The use of a monoclonal antibody and/or a derivative thereof as claimed in claim 1 for the qualitative and quantitative determination of IgE-producing cells.

34. The use as claimed in claim 33 of a monoclonal antibody and/or a derivative thereof in a plaque forming cell assay.

35. A test kit for the qualitative and quantitative determination of IgE and/or IgE-producing cells comprising a monoclonal antibody and/or a derivative thereof as claimed in claim 1 and, optionally, other monoclonal or polyclonal antibodies and/or adjuncts.

36. The use of a monoclonal antibody and/or a derivative thereof as claimed in claim 1 for the qualitative and quantitative determination of surface IgE positive (sIgE$^+$) B cells.

37. The use of a monoclonal antibody and/or a derivative thereof as claimed in claim 1 for the treatment and/or prophylaxis of allergy.

38. A pharmaceutical composition comprising a monoclonal antibody and/or a derivative thereof as claimed in claim 1.

Claims for the following Contracting State: ES

1. A process for the preparation of monoclonal antibodies directed against isotypic determinants of immunoglobulin E (IgE) which inhibit the binding of free IgE to cells bearing Fc$_\epsilon$ receptors I or II and recognize IgE expressed on the surface of IgE surface positive B cells, but do not recognize IgE bound to the surface of cells bearing Fc$_\epsilon$ receptors I or II and do not trigger mediator release from such cells, and of derivatives thereof retaining the specificity of the antibody from which they are derived, characterized in that

27

cells of a continuous cell line secreting said monoclonal antibodies are multiplied in vitro or in vivo and, when required, the obtained monoclonal antibodies are isolated and/or converted into derivatives thereof.

2. A process as claimed in claim 1 for the preparation of monoclonal antibodies and derivatives thereof which do not recognize IgE bound on the surface of $Fc_\epsilon$ receptor I bearing mast cells and basophils and do not trigger anaphylactic mediator release from such cells.

3. A process as claimed in claim 1 for the preparation of monoclonal antibodies and derivatives thereof which specifically inhibit IgE formation in the immune response.

4. A process as claimed in claim 1 for the preparation of monoclonal antibodies, which are of immunoglobulin class IgG, and of derivatives thereof.

5. A process as claimed in claim 1 for the preparation of monoclonal antibodies and derivatives thereof which are directed against isotypic determinants of murine IgE.

6. A process as claimed in claim 1 for the preparation of monoclonal rat antibodies and derivatives thereof which are directed against isotypic determinants of murine IgE.

7. A process as claimed in claim 1 for the preparation of the monoclonal antibody with the designation MAb 1-5, and of derivatives thereof.

8. A process as claimed in claim 1 for the preparation of monoclonal antibodies and derivatives thereof which are directed against isotypic determinants of human IgE.

9. A process as claimed in claim 1 for the preparation of monoclonal mouse or rat antibodies and derivatives thereof which are directed against isotypic determinants of human IgE.

10. A process as claimed in claim 1 for the preparation of monoclonal mouse antibodies and derivatives thereof which are directed against isotypic determinants of human IgE.

11. A process as claimed in claim 1 for the preparation of the monoclonal antibody with the designation MAb F1-43-17, and of derivatives thereof.

12. A process as claimed in claim 1 for the preparation of monoclonal chimeric animal/human antibodies and derivatives thereof which are directed against isotypic determinants of human IgE.

13. A process as claimed in claim 1 for the preparation of monoclonal chimeric animal/human antibodies and derivatives thereof which are directed against isotypic determinants of human IgE, and which consist of murine variable regions and human constant regions.

14. A process as claimed in claim 1 for the preparation of bispecific monoclonal antibodies comprising two different antigen binding portions, one derived from a monoclonal antibody directed against isotypic determinants of human IgE and the second derived from an antibody with a different specificity, and of derivatives thereof.

15. A process as claimed in claim 1 for the preparation of bispecific monoclonal antibodies comprising two different antigen binding portions, one derived from a monoclonal antibody directed against isotypic determinants of human IgE and the second derived from an antibody directed against a cytostatic or cytotoxic substance, and of derivatives thereof.

16. A process as claimed in claim 1 for the preparation of derivatives of monoclonal antibodies which are antibody fragments.

17. A process as claimed in claim 1 for the preparation of derivatives of monoclonal antibodies which are antibodies conjugates with an enzyme, a fluorescent marker, a chemiluminescent marker, a metal chelate, avidin, biotin, or the like.

18. A process as claimed in claim 1 for the preparation of derivatives of monoclonal antibodies which are radioactively labelled antibodies.

19. A process for the preparation of a continuous cell line which is a hybridoma cell line secreting monoclonal antibodies as defined in claim 1, characterized in that a suitable mammal is immunized with IgE, antibody-producing cells of this mammal are fused with cells of a continuous cell line, the hybrid cells obtained in the fusion are cloned, and cell clones secreting the desired monoclonal antibodies are selected.

20. A process as claimed in claim 19 for the preparation of a hybridoma cell line as claimed in claim 20 which is a hybrid of myeloma cells and B lymphocytes of a mammal immunized with IgE.

21. A process as claimed in claim 19 for the preparation of a hybridoma cell line which is a hybrid of mouse myeloma cells and B lymphocytes of a rat immunized with murine IgE.

22. A process as claimed in claim 19 for the preparation of a hybridoma cell line with the designation 654-1-5 which was deposited at the European Collection of Animal Cell Cultures under the number ECACC 89041902 on April 19,1989.

23. A process as claimed in claim 19 for the preparation of a hybridoma cell line which is a hybrid of mouse myeloma cells and B lymphocytes of a syngeneic mouse immunized with human IgE.

24. A process as claimed in claim 19 for the preparation of a hybridoma cell line with the designation F1-43-17 which was deposited at the European Collection of Animal Cell Cultures under the number

EP 0 396 505 A2

ECACC 90032210 on March 22,1990.

25. A process for the preparation of transfectoma cell lines secreting chimeric animal/human monoclonal antibodies as defined in claim 12 wherein cells of a continuous cell line are transfected with nucleic acid constructs encoding the chimeric animal/human light and/or heavy chains of the desired chimeric monoclonal antibodies, the transfected cells obtained are cloned, and cell clones secreting the desired chimeric monoclonal antibodies are selected.

26. A process for the preparation of a continuous cell line secreting bispecific monoclonal antibodies as defined in claim 14 wherein cells producing heavy and/or light chains of a monoclonal antibody directed against isotypic determinants of human IgE and cells producing heavy and/or light chains of a monoclonal antibody with a different specificity are fused, the hybrid cells obtained in the fusion are cloned, and cell clones secreting the desired bispecific monoclonal antibodies are selected.

27. A process for the qualitiative and quantitative determination of IgE wherein a monoclonal antibody and/or derivative thereof as defined in claim 1 is used.

28. A process for the qualitative and quantitative determination of surface IgE positive (sIgE$^+$) B cells wherein a monoclonal antibody and/or a derivative thereof as defined in claim 1 is used.

29. A process for the preparation of a pharmaceutical composition wherein a monoclonal antibody and/or a derivative thereof as defined in claim 1 is mixed with a pharmaceutically acceptable carrier.


Claims for the following Contracting State: GR

1. A diagnostic monoclonal antibody directed against isotypic determinants of immunoglobulin E (IgE) which inhibits the binding of free IgE to cells bearing Fc$_\epsilon$ receptors I or II and recognizes IgE expressed on the surface of IgE surface positive B cells, but does not recognize IgE bound to the surface of cells bearing Fc$_\epsilon$ receptors I or II and does not trigger mediator release from such cells, and a derivative thereof retaining the specificity of the antibody from which it is derived.

2. A monoclonal antibody as claimed in claim 1 which does not recognize IgE bound on the surface of Fc$_\epsilon$ receptor I bearing mast cells and basophils and does not trigger anaphylactic mediator release from such cells, and derivatives thereof.

3. A monoclonal antibody as claimed in claim 1 which specifically inhibits IgE formation in the immune response, and a derivative thereof.

4. A monoclonal antibody as claimed in claim 1 which is of immunoglobulin class IgG, and a derivative thereof.

5. A monoclonal antibody as claimed in claim 1 directed against isotypic determinants of murine IgE, and a derivative thereof.

6. A monoclonal antibody as claimed in claim 5 which is a rat antibody, and a derivative thereof.

7. The monoclonal antibody as claimed in claim 6 with the designation MAb 1-5, and a derivative thereof.

8. A monoclonal antibody as claimed in claim 1 directed against isotypic determinants of human IgE, and a derivative thereof.

9. A monoclonal antibody as claimed in claim 8 which is a mouse or rat antibody, and a derivative thereof.

10. A monoclonal antibody as claimed in claim 8 which is a mouse antibody, and a derivative thereof.

11. The monoclonal antibody as claimed in claim 10 with the designation MAb F1-43-17, and a derivative thereof.

12. A bispecific monoclonal antibody comprising two different antigen binding portions, one derived from a monoclonal antibody as claimed in claim 8 and the second derived from an antibody with a different specificity, and a derivative thereof.

13. A derivative of a monoclonal antibody as claimed in claim 1 which is an antibody fragment.

14. A derivative of a monoclonal antibody as claimed in claim 1 which is an antibody conjugate with an enzyme, a fluorescent marker, a chemiluminescent marker, a metal chelate, avidin, biotin, or the like.

15. A derivative of a monoclonal antibody as claimed in claim 1 which is a radioactively labelled antibody.

16. A process for the preparation of a diagnostic or therapeutic monoclonal antibody directed against isotypic determinants of immunoglobulin E (IgE) which inhibits the binding of free IgE to cells bearing Fc$_\epsilon$ receptors I or II and recognizes IgE expressed on the surface of IgE surface positive B cells, but does not recognize IgE bound to the surface of cells bearing Fc$_\epsilon$ receptors I or II and does not trigger mediator release from such cells, and of a derivative thereof retaining the specificity of the antibody from which it is derived, wherein cells of a continuous cell line secreting said monoclonal antibodies are multiplied in vitro or

29

in vivo and, when required, the obtained monoclonal antibody is isolated and/or converted into a derivative thereof.

17. A process as claimed in claim 16 for the preparation of a chimeric animal/human antibody directed against isotypic determinants IgE, and of a derivative thereof.

18. A process as claimed in claim 16 for the preparation of a chimeric animal/human antibody directed against isotypic determinants IgE, which consists of murine variable regions and human constant regions, and of a derivative thereof.

19. A process as claimed in claim 16 for the preparation of a bispecific monoclonal antibody comprising two different antigen binding portions, one derived from a monoclonal antibody as claimed in claim 8 and the second derived from an antibody directed against a cytostatic or cytotoxic substance, and of a derivative thereof.

20. A continuous cell line which is a hybridoma cell line secreting monoclonal antibodies directed against isotypic determinants of immunoglobulin E (IgE) which inhibits the binding of free IgE to cells bearing $Fc_\epsilon$ receptors I or II and recognizes IgE expressed on the surface of IgE surface positive B cells, but does not recognize IgE bound to the surface of cells bearing $Fc_\epsilon$ receptors I or II and does not trigger mediator release from such cells.

21. A hybridoma cell line as claimed in claim 20 which is a hybrid of myeloma cells and B lymphocytes of a mammal immunized with IgE.

22. A hybridoma cell line as claimed in claim 20 which is a hybrid of mouse myeloma cells and B lymphocytes of a rat immunized with murine IgE.

23. The hybridoma cell line as claimed in claim 22 with the designation 654-1-5 which was deposited at the European Collection of Animal Cell Cultures under the number ECACC 89041902 on April 19,1989.

24. A hybridoma cell line as claimed in claim 20 which is a hybrid of mouse myeloma cells and B lymphocytes of a syngeneic mouse immunized with human IgE.

25. The hybridoma cell line as claimed in claim 24 with the designation F1-43-17 which was deposited at the European Collection of Animal Cell Cultures under the number ECACC 90032210 on March 22, 1990.

26. A process for the preparation of hybridoma cell lines as claimed in claim 20 wherein a suitable mammal is immunized with IgE, antibody-producing cells of this mammal are fused with cells of a continuous cell line, the hybrid cells obtained in the fusion are cloned, and cell clones secreting the desired monoclonal antibodies are selected.

27. A continuous cell line which is a transfectoma cell line secreting chimeric animal/human monoclonal antibodies as defined in claim 17.

28. A process for the preparation of transfectoma cell lines as claimed in claim 27 wherein cells of a continuous cell line are transfected with nucleic acid constructs encoding the chimeric animal/human light and/or heavy chains of the desired chimeric monoclonal antibodies, the transfected cells obtained are cloned, and cell clones secreting the desired chimeric monoclonal antibodies are selected.

29. A continuous cell line secreting bispecific monoclonal antibodies as claimed in claim 12.

30. A process for the preparation of a continuous cell line as claimed in claim 29 wherein cells producing heavy and/or light chains of a monoclonal antibody directed against isotypic determinants of human IgE and cells producing heavy and/or light chains of a monoclonal antibody with a different specificity are fused, the hybrid cells obtained in the fusion are cloned, and cell clones secreting the desired bispecific monoclonal antibodies are selected.

31. The use of a monoclonal antibody and/or derivative thereof as claimed in claim 1 for the qualitiative and quantitative determination of IgE.

32. The use as claimed in claim 31 of a monoclonal antibody and/or derivative thereof in a radioimmunoassay, an enzyme immunoassay or a chemiluminescence immunoassay.

33. The use of a monoclonal antibody and/or a derivative thereof as claimed in claim 1 for the qualitative and quantitative determination of IgE-producing cells.

34. The use as claimed in claim 33 of a monoclonal antibody and/or a derivative thereof in a plaque forming cell assay.

35. A test kit for the qualitative and quantitative determination of IgE and/or IgE-producing cells comprising a monoclonal antibody and/or a derivative thereof as claimed in claim 1 and, optionally, other monoclonal or polyclonal antibodies and/or adjuncts.

36. The use of a monoclonal antibody and/or a derivative thereof as claimed in claim 1 for the qualitative and quantitative determination of surface IgE positive ($sIgE^+$) B cells.

37. A process for the preparation of a pharmaceutical composition wherein a monoclonal antibody and/or a derivative thereof as defined in claim 16 is mixed with a pharmaaceutically acceptable carrier.